# EUROPEAN PATENT APPLICATION

(11) **EP 4 302 749 A1**
(43) Date of publication of application: **10.01.2024**
(21) Application number: 22183270.2
(22) Date of filing: 06.07.2022
(51) Int. Cl.: A61K 9/00, A61K 9/08, A61K 38/00, G01N 33/00, A61K 47/42

(54) **PHYSIOLOGICAL SALT SOLUTION AND METHOD FOR DETERMINING BIOEQUIVALENCE**

(71) Applicant: Joanneum Research Forschungsgesellschaft mbH, 8010 Graz (AT)
(72) Inventor: BIRNGRUBER, Thomas, 8010 Graz (AT); HUMMER, Joana, 8051 Graz (AT); RAML, Reingard, 8047 Hart bei Graz (AT)
(74) Representative: Geling, Andrea

(57) **Abstract**

The present invention relates to a physiological salt solution comprising serum albumin. Further, the present invention relates to the use of the physiological salt solution as dialysis solution for microdialysis (MD), or perfusion solution for Open Flow Microperfusion (OFM). Furthermore, the present invention relates to a kit for microdialysis (MD) or Open Flow Microperfusion (OFM), wherein said kit comprises the physiological salt solution. In addition, the present invention relates to a method for determining bioequivalence between a test substance and a reference substance. Moreover, the present invention relates to a method for determining bioequivalence between a first treatment site of an individual treated with a test substance and a second treatment site of the individual treated with the (same) test substance.

## Description

The present invention relates to a physiological salt solution comprising serum albumin. Further, the present invention relates to the use of the physiological salt solution as dialysis solution for microdialysis (MD), or perfusion solution for Open Flow Microperfusion (OFM). Furthermore, the present invention relates to a kit for microdialysis (MD) or Open Flow Microperfusion (OFM), wherein said kit comprises the physiological salt solution. In addition, the present invention relates to a method for determining bioequivalence between a test substance and a reference substance. Moreover, the present invention relates to a method for determining bioequivalence between a first treatment site of an individual treated with a test substance and a second treatment site of the individual treated with the (same) test substance.

### BACKGROUND OF THE INVENTION

In many countries, strategies to control healthcare spending rely upon the availability and use of generic medicines. The safety and effectiveness of high-quality generic medicines is ensured through a demonstration of bioequivalence (BE). The methodology for assessing the BE of systemically absorbed drugs and for the statistical assessment of comparative systemic bioavailability (BA) based upon pharmacokinetic (PK) endpoints for drugs that were transported via the blood circulation (oral, infusion, subcutaneous injection) is well established.

This does not apply for topical drug products, which are not intended to be absorbed into the systemic circulation (blood). The BE testing of topical dermal products requires in general, a randomized, double-blind, parallel, placebo-controlled, comparative, clinical endpoint BE study that compares the test (generic) product with the reference listed drug product.

Recently, dermal open-flow microperfusion (dOFM) has been identified as a potential approach to assess the BA/BE of topical products. It facilitates a continuous assessment of the *in vivo* cutaneous kinetics of topically administered drugs directly in the dermis in human subjects. dOFM can assess the intradermal drug concentrations by sampling the dermal interstitial fluid for up to 48 h. The dOFM approach allows a direct PK approach for BE assessment in the dermis. In addition, dOFM allows a direct comparison of two different topical drugs in the same subject and avoids therefore high intersubject variability. It is possible to perform the same setup with microdialysis (MD), specifically dermal microdialysis (dMD).

Continuous sampling systems like dOFM or dMD rely on a liquid medium that is circulated through the probes and acts as a transport medium, the perfusion or dialysis solution. After dOFM or dMD, the obtained perfusate or diffusate is finally analyzed. The composition of the perfusion or dialysis solution influences the degree of extraction of the substance that is being examined. The basic physical principals behind this effect are ionic strength, pH-value, osmotic, colloid osmotic and protein binding effects. The ideal perfusion or dialysis solution matches the dermal interstitial fluid in order to avoid complex exchange processes at the exchange area of dOFM and dMD. Such an ideal perfusion or dialysis solution is currently not available.

Thus, there is an unmet need for the provision of a physiological salt solution that may be used for dOFM or dMD and that ideally matches the dermal interstitial fluid.

The present inventors were surprisingly able provide such a physiological salt solution. This solution can be used in dOFM and dMD experiments in order to avoid distortion of the results due to varying or inadequate compositions of the perfusion or dialysis solution.

The present inventors have further established a method for determining bioequivalence between a test substance and a reference substance and a method for determining bioequivalence between a first treatment site of an individual treated with a test substance and a second treatment site of the individual treated with the (same) test substance. In these methods, the proprietary physiological salt solution is used.

### SUMMARY OF THE INVENTION

In a first aspect, the present invention relates to a physiological salt solution comprising serum albumin.

In a second aspect, the present invention relates to the use of the physiological salt solution of the first aspect as dialysis solution for microdialysis (MD), or perfusion solution for Open Flow Microperfusion (OFM).

In a third aspect, the present invention relates to a kit for microdialysis (MD) or Open Flow Microperfusion (OFM), said kit comprises:
(i) a physiological salt solution of the first aspect, and
(ii) a dialysis or perfusion tube.

In a fourth aspect, the present invention relates to a method for determining bioequivalence between a test substance and a reference substance comprising the steps of:
(i) providing at least one dialysate or perfusate comprising a test substance obtained from an individual treated on its skin with the test substance,
(ii) providing at least one dialysate or perfusate comprising a reference substance obtained from the individual treated on its skin with the reference substance,
(iii) determining at least one pharmacokinetic parameter of the test substance in the at least one dialysate or perfusate provided in (i) and determining (the same) at least one pharmacokinetic parameter of the reference substance in the at least one dialysate or perfusate provided in (ii), and
(iv) comparing the at least one pharmacokinetic parameter of the test substance with the (same) at least one pharmacokinetic parameter of the reference substance,
   wherein an equivalence/a comparability between the at least one pharmacokinetic parameter of the test substance with the (same) at least one pharmacokinetic parameter of the reference substance indicates bioequivalence of the test substance and the reference substance.

In a fifth aspect, the present invention relates to a method for determining bioequivalence between a test substance and a reference substance using a kit of the third aspect.

In a sixth aspect, the present invention relates to a method for determining bioequivalence between a first treatment site of an individual treated with a test substance and a second treatment site of the individual treated with the (same) test substance comprising the steps of:
(i) providing at least one dialysate or perfusate comprising a test substance obtained from an individual treated on its skin at a first treatment site with the test substance,
(ii) providing at least one dialysate or perfusate comprising the (same) test substance obtained from the individual treated on its skin at a second treatment site with the (same) test substance,
(iii) determining at least one pharmacokinetic parameter of the test substance at a first treatment site in the at least one dialysate or perfusate provided in (i) and determining (the same) at least one pharmacokinetic parameter of the (same) test substance at the second treatment site in the at least one dialysate or perfusate provided in (ii), and
(iv) comparing the at least one pharmacokinetic parameter of the test substance at the first treatment site with the (same) at least one pharmacokinetic parameter of the (same) test substance at the second treatment site,
   wherein an equivalence/a comparability between the at least one pharmacokinetic parameter of the test substance at the first treatment site with the (same) at least one pharmacokinetic parameter of the (same) test substance at the second treatment site indicates bioequivalence of the first treatment site of the individual treated with the test substance and the second treatment site of the individual treated with the (same) test substance.

In a seventh aspect, the present invention relates to a method for determining bioequivalence between a first treatment site of an individual treated with a test substance and a second treatment site of the individual treated with the (same) test substance using a kit of the third aspect.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

As used herein, the term "about" indicates a certain variation from the quantitative value it precedes. In particular, the term "about" allows a ±5% variation from the quantitative value it precedes, unless otherwise indicated or inferred. The use of the term "about" also includes the specific quantitative value itself, unless explicitly stated otherwise. For example, the expression "about 80°C" allows a variation of ±4°C, thus referring to range from 76°C to 84°C.

Strategies to control healthcare spending rely upon the availability and use of generic medicines. The safety and effectiveness of high-quality generic medicines is ensured through a demonstration of bioequivalence (BE).

The United States Food and Drug Administration (FDA) recognizes the public policy benefit of generic competition but has understandably embraced safeguards to protect the public from generics that do not meet quality standards and/or are not objectively bioequivalent to the brand-name product. To be allowed by the FDA, it has to be demonstrated that the generic drug product has the same active ingredient, route of administration, dosage form and strength, and proposed labeling as the brand-name drug. In addition, it has to be demonstrated that the generic drug is "bioequivalent" to the relevant brand-name product. When acceptable information of this type is provided, the generic applicant is permitted to rely on the FDA's previous findings of safety and effectiveness for the referenced brand-name drug, and thus, in theory, the sponsor of the generic does not have to provide its own clinical studies to demonstrate the generic drug product's safety and effectiveness.

The physiological salt solution of the present invention can be used in such bioequivalence (BE) analysis and, thus, allows to determine whether the generic drug product (test substance) is bioequivalent to the brand-name product (reference substance) and can alternatively be used.

The term "physiological salt solution", as described herein, refers to a solution comprising serum albumin. As the name already says, the physiological salt solution further comprises (a mixture of) water and salt(s). The physiological salt solution is essentially isotonic with tissue fluids or blood. It is pharmaceutically and dermatologically acceptable and has a pH in physiological range and/or an osmolarity in physiological range.

Preferably, the physiological salt solution has a physiological pH, i.e. a pH of between 7.35 to 7.45, with the average at 7.40, and/or an osmolarity in a range of between 300 and 310 mOsm/l. The physiological salt solution is suitable for medical applications, e.g. in order to determine bioequivalence of a drug. In addition, the physiological salt solution is sterile. It is used as dialysis solution for bioequivalence studies with microdialysis (MD), or as perfusion solution for bioequivalence studies with Open Flow Microperfusion (OFM).

"Bioequivalence", as used herein, is a term in pharmacokinetics used to assess the expected *in vivo* biological equivalence of two proprietary preparations of a drug (e.g. test substance and reference substance). If two products are said to be bioequivalent it means that they would be expected to be, for all intents and purposes, the same.

Specifically, two pharmaceutical products are bioequivalent if they are pharmaceutically equivalent or pharmaceutical alternatives and their bioavailabilities, in terms of rate (Cmax and tmax) and extent of absorption (area under the curve), after administration of the same molar dose under the same conditions, are similar to such a degree that their effects can be expected to be essentially the same.

Defined in a different way, bioequivalence is the absence of a significant difference in the rate and extent to which the active ingredient or active moiety in pharmaceutical equivalents or pharmaceutical alternatives becomes available at the site of drug action when administered at the same molar dose under similar conditions in an appropriately designed study.

It is preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 70% to 135%, e.g. 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, or 135%. It is more preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 75% to 130%, e.g. 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130%.

It is even more preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 80% to 125%, e.g. 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, or 125%.

In general, to determine bioequivalence between two products such as a potential to-be-marketed generic product (test substance) and a commercially-available brand-name product (reference substance), pharmacokinetic studies are conducted whereby each of the preparations are administered in a cross-over study to volunteer subjects, generally healthy individuals but occasionally in patients.

In the method described herein, bioequivalence between a test substance and a reference substance is determined on the same subject.

In an alternative method, bioequivalence between a first treatment site of a subject treated with a test substance and a second treatment site of the subject treated with the (same) test substance is determined.

Described herein is specifically the determination of the bioequivalence of an active pharmaceutical ingredient (API) or dermal drug.

The term "bioavailability" as used herein refers to the rate and extent to which a drug (test substance) relative to a reference brand-name drug (reference substance) becomes available at the site of action. For topical/dermal drugs, the site of action is usually the lower epidermis. However, transdermal administration has a barrier, the epidermal/dermal drug barrier. Particularly, the *stratum corneum* (SC), the top layer of the skin, provides the main barrier to the skin. The skin wall contains lipid bilayers through which drugs must pass. The SC barrier is not easy to overcome. For example, the permeability of the intestine wall is much higher compared to the SC in the skin. There have always been challenges to delivering the therapeutic amounts of drugs to targets. Bioavailability in dermal drugs delivery is mostly limited by instability of the compound and by the skin barrier.

Described herein is specifically the determination of the bioavailability of an active pharmaceutical ingredient (API) or dermal drug.

In the methods of the present invention, pharmacokinetic parameters for determining bioequivalence are measured. "Pharmacokinetics" (abbreviated as "PK"), as used herein, is to determine the fate of substances administered externally to a living organism. In practice, a PK study is applied mainly to drug substances, though in principle it concerns itself with all manner of compounds delivered externally to an organism. Pharmacokinetics is often studied in conjunction with pharmacodynamics. Pharmacodynamics explores what a drug does to the body, whereas pharmacokinetics explores what the body does to the drug. Pharmacokinetics includes the study of the mechanisms of absorption and distribution of an administered drug, the rate at which a drug action begins and the duration of the effect, the chemical changes of the substance in the body (e.g. metabolism by enzymes) and the effects and routes of excretion of the metabolites of the drug. Pharmacokinetics is divided into several areas which include the extent and rate of absorption, distribution, metabolism and excretion. Pharmacokinetics describes how the body affects a specific drug after administration. Pharmacokinetic properties of drugs may be affected by elements such as the site of administration, the concentration in which the drug is administered and the dosage form (immediate or controlled release for example). These may affect the absorption rate. An important pharmacokinetic parameter is the biological half-life or elimination half-life of a substance, which is the time it takes for a substance to lose half of its pharmacologic or physiologic activity.

For a pharmacokinetic comparison, at least one pharmacokinetic parameter selected from the group consisting of absorption rate constant, bioavailability, apparent volume of distribution, steady-state volume of distribution, unbound fraction, rate of elimination, clearance, renal clearance, metabolic clearance, fraction excreted unchanged, elimination rate constant, biologic half-life, maximum concentration, area under the concentration vs. time curve (AUC) and toxicity, preferably dermal toxicity, is assessed.

Described herein is specifically the determination of the pharmacokinetics of an active pharmaceutical ingredient (API) or dermal drug.

The term "microdialysis (MD)", as used herein, refers to a minimally-invasive sampling technique that is used for continuous measurement of free, unbound analyte concentrations in the extracellular fluid of virtually any tissue. Analytes may include endogenous molecules (e.g. hormones) to assess their biochemical functions in the body, or exogenous compounds (e.g. drugs) to determine their distribution within the body. The microdialysis technique requires the insertion of a small dialysis catheter (also referred to as dialysis tube) into the tissue of interest. The dialysis tube is designed to mimic a capillary and consists of a shaft with a semipermeable hollow fiber membrane at its tip, which is connected to inlet and outlet tubing. The probe is continuously streamed with an aqueous solution (dialysis solution) that closely resembles the (ionic) composition of the surrounding tissue fluid at a low flow rate. This solution is preferably the physiological salt solution as described herein. Once inserted into the tissue or (body)fluid of interest, small solutes can cross the semipermeable membrane by passive diffusion. The direction of the analyte flow is determined by the respective concentration gradient and allows the usage of microdialysis probes as sampling as well as delivery tools. The solution leaving the probe (dialysate) is collected at certain time intervals for analysis.

Described herein is specifically the microdialysis (MD) of the skin. The drug is preferably an active pharmaceutical ingredient (API) or dermal drug.

The term "dermal microdialysis (dMD)", as used herein, is a versatile sampling technique that can be used to recover soluble endogenous and exogenous molecules from the extracellular compartment of the dermis. Due to its minimally invasive character, dermal microdialysis can be applied in both clinical and preclinical settings.

To perform dermal microdialysis dialysis tubes having a lumen and a semipermeable membrane are inserted into the dermis or the subcutis and perfused at a low speed with a physiological salt solution (dialysis solution). Endogenous or exogenous molecules soluble in the extracellular fluid, specifically intestinal dermal fluid, diffuse into the dialysis tube and are collected in small vials for analysis. The duration and timing of the collected dialysate samples allows kinetic evaluation of the events occurring in the tissue.

Specific placement of probes into the dermal layer opens the way for studies of inflammatory events most prominently driven by that part of the skin. Dermal microdialysis can also be applied in drug discovery or pharmacokinetic/pharmacodynamic (PK/PD) studies and in the study of percutaneous penetration of potentially harmful exogenous agents from the environment.

In addition, dermal microdialysis can be applied for bioequivalence studies. In this case, the dialysis tube allows the diffusion of the drug (test substance) or reference brand-name drug (reference substance) from the dermal intestinal fluid to a dialysis solution comprised in its lumen. The dialysis solution is preferably a physiological salt solution as disclosed herein. The dialysate is then further analyzed.

The term "Open Flow Microperfusion (OFM)", as used herein, refers to a probe-based method that is used to evaluate the pharmacokinetics and pharmacodynamics of drugs directly in tissue. The use of the OFM probes allows small amounts of extracellular fluid to be collected from target tissues for analysis.

The Open Flow Microperfusion technique requires the insertion of a small perfusion catheter (also referred to as perfusion tube) into the tissue of interest. The perfusion tube is designed to mimic a capillary and consists of a shaft with a permeable hollow fiber membrane at its tip, which is connected to inlet and outlet tubing.

The probe is continuously streamed with an aqueous solution (perfusion solution) that closely resembles the (ionic) composition of the surrounding tissue fluid at a low flow rate. This solution is preferably the physiological salt solution as described herein. Once inserted into the tissue or (body)fluid of interest, small solutes can cross the permeable membrane by passive diffusion. The solution leaving the probe (perfusate) is collected at certain time intervals for analysis. Described herein is specifically the Open Flow Microperfusion (OFM) of the skin. The drug is preferably an active pharmaceutical ingredient (API) or dermal drug.

The term "dermal Open Flow Microperfusion (dOFM)", as used herein, is a versatile sampling technique that allows the investigation of transport of drugs in the dermis and their penetration into the dermis after local, topical or systemic application. Dermal Open Flow Microperfusion is used for the conduction of tissue-specific pharmacokinetic (PK) and pharmacodynamic (PD) studies of drugs, for the assessment of dermal bioavailability, or for the evaluation of topical generic products, which need to demonstrate bioequivalence to the reference listed drug product to obtain market approval. In particular, dOFM facilitates a continuous assessment of the *in vivo* cutaneous kinetics of topically administered drugs directly in the dermis in human subjects. dOFM can assess the intradermal drug concentrations by sampling the dermal interstitial fluid for up to 48 h. The dOFM approach allows a direct PK approach for BE assessment in the dermis. In addition, dOFM allows a direct comparison of two different topical drugs in the same subject and avoids therefore high intersubject variability.

As mentioned above, dOFM can be applied for bioequivalence studies. In this case, the perfusion tube allows the perfusion of the drug (test substance) or reference brand-name drug (reference substance) from the dermal intestinal fluid to a perfusion solution comprised in its lumen. The perfusion solution is preferably a physiological salt solution as disclosed herein. The perfusate is then further analyzed.

In this context, it should be noted that the main difference between dMD and dOFM lies in the tissue interface. While dMD uses a semipermeable membrane, dOFM uses an exchange area with macroscopic holes.

The term "dialysate", as used herein, refers to the liquid obtained from/being the result of microdialysis, particularly dermal microdialysis (dMD). The dialysate described herein comprises a test substance or reference substance obtained from an individual treated on its skin with the test substance or reference substance. The dialysate is obtained from the individual via a dialysis tube embedded/inserted into the tissue underlying the skin of said individual. The dialysis tube comprises a lumen and a semipermeable membrane. The test substance or reference substance applied to the skin penetrates the skin and enters the dermal interstitial fluid that surrounds cells and tissue underlying the skin. The dialysis tube allows the diffusion of the test substance or reference substance from the dermal intestinal fluid (via the semi-permeable membrane) into a dialysis solution comprised in its lumen. It is preferred that the dialysis solution is the physiological salt solution as described herein.

Thus, the dialysate comprising a test substance or reference substance further comprises the dialysis solution, preferably the physiological salt solution as described herein, and dermal interstitial fluid.

The term "perfusate", as used herein, refers to the liquid obtained from/being the result of Open Flow Microperfusion (OFM), particularly dermal Open Flow Microperfusion (dOFM). The perfusate described herein comprises a test substance or reference substance obtained from an individual treated on its skin with the test substance or reference substance. The perfusate is obtained from the individual via a perfusion tube embedded/inserted into the tissue underlying the skin of said individual. The perfusion tube comprises a lumen and a permeable membrane. The test substance or reference substance applied to the skin penetrates the skin and enters the dermal interstitial fluid that surrounds cells and tissue underlying the skin. The perfusion tube allows the diffusion of the test substance or reference substance from the dermal intestinal fluid (via the permeable membrane) into a perfusion solution comprised in its lumen. It is preferred that the perfusion solution is the physiological salt solution as described herein.

Thus, the perfusate comprising a test substance or reference substance further comprises the perfusion solution, preferably the physiological salt solution as described herein, and dermal interstitial fluid.

The term "intestinal fluid (ISF)", as used herein, refers to a fluid that forms the immediate environment of all cells. In other words, the term "intestinal fluid (ISF) refers to a fluid found in the spaces around cells. It comes from substances that leak out of blood capillaries (the smallest type of blood vessel). It helps bring oxygen and nutrients to cells and to remove waste products from them. As new interstitial fluid is made, it replaces older fluid, which drains towards lymph vessels. When it enters the lymph vessels, it is called lymph. Also called tissue fluid. The interstitial fluid consists of a water solvent containing amino acids, sugars, fatty acids, coenzymes, hormones, neurotransmitters, salts as well as waste products from the cells. The composition of intestinal fluid depends upon the exchanges between the cells in the tissue and the blood. This means that tissue fluid has a different composition in different tissues and in different areas of the body. Not all of the contents of the blood pass into the tissue, which means that tissue fluid and blood are not the same. Red blood cells, platelets, and plasma proteins cannot pass through the walls of the capillaries. The resulting mixture that does pass through is essentially blood plasma without the plasma proteins. Tissue fluid also contains some types of white blood cells which help combat infection. Lymph is considered a part of the interstitial fluid. The lymphatic system returns protein and excess interstitial fluid to the circulation.

The term "dermal intestinal fluid (dISF)", as used herein, refers to a fluid that forms the immediate environment of all cells in the dermis. In other words, the term "dermal intestinal fluid (dISF) refers to a fluid found in the spaces around dermal cells.

The term "test substance", as used herein, refers to a substance or mixture administered or added to a test system in a study, which substance or mixture is used to develop data. In the context of the present invention, pharmacokinetic parameters of a test substance are determined. In the context of the present invention, the test substance is applied to the skin/dermis of a subject. Preferably, the test substance is an active pharmaceutical ingredient (API) or a dermal drug. More preferably, the test substance is a generic drug, particularly a generic dermal drug.

The term "reference substance", as used herein, refers to a reference substance or mixture administered or added to a test system in a study, which is other than the test substance for the purpose of establishing a basis for comparison with the test substance for known chemical or biological measurements. In the context of the present invention, pharmacokinetic parameters of a reference substance are determined. In the context of the present invention, the reference substance is applied to the skin/dermis of a subject. Preferably, the reference substance is an active pharmaceutical ingredient (API) or a dermal drug.

More preferably, the test substance is a reference brand-name drug, particularly a reference brand-name dermal drug.

As mentioned above, the United States Food and Drug Administration (FDA) recognizes the public policy benefit of generic competition but has understandably embraced safeguards to protect the public from generics that do not meet quality standards and/or are not objectively bioequivalent to the brand-name product. To be allowed by the FDA, it has to be demonstrated that the generic drug product (test substance) has the same active ingredient, route of administration, dosage form and strength, and proposed labeling as the brand-name drug (reference substance). In addition, it has to be demonstrated that the generic drug is "bioequivalent" to the relevant brand-name product. When acceptable information of this type is provided, the generic applicant is permitted to rely on the FDA's previous findings of safety and effectiveness for the referenced brand-name drug, and thus, in theory, the sponsor of the generic does not have to provide its own clinical studies to demonstrate the generic drug product's safety and effectiveness.

The term "generic drug", as used herein, refers to a pharmaceutical drug that contains the same chemical substance as a drug that was protected by chemical patents. Generic drugs are allowed for sale after the patents on the original drugs expire. Because the active chemical substance is the same, the medical profile of generics is believed to be equivalent in performance. A generic drug has the same active pharmaceutical ingredient (AIP) as the original, but it may differ in some characteristics such as the manufacturing process, formulation, excipients, colour, taste, and packaging.

Although they may not be associated with a particular company, generic drugs are usually subject to government regulations in the countries in which they are dispensed. They are labelled with the name of the manufacturer and a generic non-proprietary name of the drug. A generic drug must contain the same active ingredient as the original brand-name formulation. The U.S. Food and Drug Administration (FDA) requires generics to be identical to or within an acceptable bioequivalent range of their brand-name counterparts, with respect to pharmacokinetic and/or pharmadynamic properties.

In a preferred embodiment, the physiological salt solution of the present invention comprises deuterated water as solvent.

The term "deuterated water" (also designated as "²H₂O enriched water"), as used herein, refers to a form of water that contains more ²H (also designated as deuterium or D, also known as heavy hydrogen) than usually present therein. Generally, common ¹H isotope (also designated as hydrogen-1 isotope or H, also known as protium) makes up most of the hydrogen in normal water (H₂O). The presence of the heavier hydrogen isotope gives the water an increased molecular mass with nearly the same physical and chemical properties when compared to normal water. In a preferred embodiment, ²H₂O enriched water contains between 0.02 % and 100% ²H₂O (specified as ²H fraction). For comparison, in the isotopic reference standard VSMOW (Vienna Standard Mean Ocean Water) only about 0.0156% of the hydrogen atoms are of the heavy type.

The term "deuterium", as used herein, refers to a hydrogen isotope with a nucleus containing a neutron and a proton; the nucleus of a protium (normal hydrogen) atom consists of just a proton. The additional neutron makes a deuterium atom roughly twice as heavy as a protium atom. A molecule of heavy water has two deuterium atoms in place of the two protium atoms of ordinary "light" water. The weight of a heavy water molecule, however, is not substantially different from that of a normal water molecule, because about 89% of the molecular weight of water comes from the single oxygen atom rather than the two hydrogen atoms.

Deuterated water is used herein as a marker which allows to determine the exchange rate between the perfusion or dialysis solution (preferably the physiological salt solution as described herein) and the dermal intestinal fluid (dISF).

The term "individual", as used herein, refers to any individual who can participate in the bioequivalence studies described herein, i.e. in the methods of the present invention.

The individual may be a volunteer subject. The individual may be a healthy individual. The individual may also be diseased, e.g. suffering from a dermal disease. Preferably, the individual is a mammal like a human such as a healthy human or diseased human, e.g. suffering from a dermal disease.

In the context of the present invention, the term "kit of parts (in short: kit)" is understood to be any combination of at least some of the components identified herein, which are combined, coexisting spatially, to a functional unit, and which can contain further components.

### Embodiments of the invention

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

As mentioned above, continuous sampling systems like dermal Open Flow Microperfusion (dOFM) or dermal microdialysis (dMD) rely on a liquid medium that is circulated through the probes and acts as a transport medium, the perfusion or dialysis solution. After dOFM or dMD, the obtained perfusate or diffusate is finally analyzed. The composition of the perfusion or dialysis solution influences the degree of extraction of the substance that is being examined. The basic physical principals behind this effect are ionic strength, pH-value, osmotic, colloid osmotic and protein binding effects. The ideal perfusion or dialysis solution matches the dermal interstitial fluid in order to avoid complex exchange processes at the exchange area of dOFM and dMD

Such an ideal perfusion or dialysis solution is currently not available. Thus, there is an unmet need for the provision of a physiological salt solution that matches the dermal interstitial fluid.

The present inventors were surprisingly able provide such a physiological salt solution. This solution can be used in dOFM and dMD experiments in order to avoid distortion of the results due to varying or inadequate compositions of the perfusion or dialysis solution.

The present inventors have further established a method for determining bioequivalence between a test substance and a reference substance and a method for determining bioequivalence between a first treatment site of an individual treated with a test substance and a second treatment site of the individual treated with the (same) test substance. In these methods, the physiological salt solution is used.

Thus, in a first aspect, the present invention relates a physiological salt solution comprising serum albumin.

Preferably, serum albumin is comprised in a concentration of between 10 and 30 g/l, e.g. 10, 11, 12, 13, 14, 15, 16, 17, 18, 19, 20, 21, 22, 23, 24, 25, 26, 27, 28, 29, or 30 g/l. More preferably, serum albumin is comprised in a concentration of between 15 and 25 g/l, e.g. 15, 16, 17, 18, 19, 20, 21, 22, 23, 24, or 25 g/l. Thus, in one embodiment, the present invention relates to a physiological salt solution comprising serum albumin, wherein the serum albumin is comprised in a concentration of between 10 and 30 g/l. Even more preferably, serum albumin is comprised in a concentration of between 18 and 22 g/l, e.g. 18, 19, 20, 21, or 22 g/l. The above-mentioned serum albumin is particularly human serum albumin.

As the name already says, the physiological salt solution comprises, in addition to serum albumin, (a mixture of) water and salt(s). The physiological salt solution is essentially isotonic with tissue fluids or blood. It is pharmaceutically and dermatologically acceptable and has a pH in physiological range and/or an osmolarity in physiological range.

Preferably, the physiological salt solution has a pH of between 7.35 to 7.45, with the average at 7.40, and/or an osmolarity in a range of between 300 and 310 mOsm/l, specifically of 302 mOsm/l. The physiological salt solution is suitable for medical applications, e.g. in order to determine bioequivalence of a test substance (e.g. a generic drug) compared to a reference substance (e.g. a brand-name drug). In addition, the physiological salt solution is sterile.

In one embodiment, the salt solution further comprises alpha-1-acid glycoprotein. Preferably, alpha-1-acid glycoprotein is comprised in a concentration of between 0.005 and 0.4 g/l, e.g. 0.005, 0.006, 0.007, 0.008, 0.009, 0.01, 0.02, 0.03, 0.04, 0.05, 0.06, 0.07, 0.08, 0.09, 0.1, 0.2, 0.3, or 0.4 g/l. More preferably, alpha-1-acid glycoprotein is comprised in a concentration of between 0.005 and 0.1 g/l, e.g. 0.005, 0.006, 0.007, 0.008, 0.009, 0.01, 0.02, 0.03, 0.04, 0.05, 0.06, 0.07, 0.08, 0.09, or 0.1 g/l. Even more preferably, alpha-1-acid glycoprotein is comprised in a concentration of between 0.005 and 0.03 g/l, e.g. 0.005, 0.006, 0.007, 0.008, 0.009, 0.01, 0.02, or 0.03 g/l.

In one another embodiment, the salt solution further comprises immunoglobulin G. Preferably, immunoglobulin G is comprised in a concentration of between 1x10⁻⁷ and 4x10⁻⁴ g/l. More preferably, immunoglobulin G is comprised in a concentration of between 2x10⁻⁷ and 2x10⁻⁴ g/l. Even more preferably, immunoglobulin G is comprised in a concentration of between 5x10⁻⁷ and 5x10⁻⁵ g/l.

In one another embodiment, the salt solution further comprises deuterated water (as solvent).

Preferably, deuterated water is comprised in a concentration of between 0.15 and 500 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, or 500 ml/l. More preferably, deuterated water is comprised in a concentration of between 0.15 and 30 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, or 30 ml/l. Even more preferably, deuterated water is comprised in a concentration of between 0.15 and 15 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, or 15 ml/l.

Instead of deuterated water, urea can be used. Preferably, urea is comprised in a concentration of between 0.18 and 5 g/l, e.g. 0.18, 0.19, 0.2, 0.3, 0.4, 0.5, 0.6, 0.7, 0.8, 0.9, 1, 2, 3, 4, or 5 g/l. More preferably, urea is comprised in a concentration of between 0.3 and 2 g/l, e.g. 0.3, 0.4, 0.5, 0.6, 0.7, 0.8, 0.9, 1, or 2 g/l.

In one preferred embodiment, the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water.
The above-mentioned serum albumin is particularly human serum albumin.
Instead of deuterated water, urea can be used.
In one particularly preferred embodiment, the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water,
   and wherein
serum albumin is comprised in a concentration of between 10 and 30 g/l, e.g. 10, 11, 12, 13, 14, 15, 16, 17, 18, 19, 20, 21, 22, 23, 24, 25, 26, 27, 28, 29, or 30 g/l,
alpha-1-acid glycoprotein (if present) is comprised in a concentration of between 0.005 and 0.4 g/l, e.g. 0.005, 0.006, 0.007, 0.008, 0.009, 0.01, 0.02, 0.03, 0.04, 0.05, 0.06, 0.07, 0.08, 0.09, 0.1, 0.2, 0.3, or 0.4 g/l,
immunoglobulin G (if present) is comprised in a concentration of between 1×10⁻⁷ and 4x10⁻⁴ g/l, and/or
deuterated water (if present) is comprised in a concentration of between 0.15 and 500 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, or 500 ml/l.
The above-mentioned serum albumin is particularly human serum albumin.

Instead of deuterated water, urea can be used. Preferably, urea is comprised in a concentration of between 0.18 and 5 g/l, e.g. 0.18, 0.19, 0.2, 0.3, 0.4, 0.5, 0.6, 0.7, 0.8, 0.9, 1, 2, 3, 4, or 5 g/l. More preferably, urea is comprised in a concentration of between 0.3 and 2 g/l, e.g. 0.3, 0.4, 0.5, 0.6, 0.7, 0.8, 0.9, 1, or 2 g/l.

In one more preferred embodiment, the salt solution further comprises one or more salts selected from the group consisting of sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), and sodium caprylate. -

In one particularly more preferred embodiment, the salt solution further comprises one or more salts selected from the group consisting of sodium chloride (NaCI), potassium chloride (KCl), calcium chloride dihydrate (CaCl₂ ·2H₂O), magnesium chloride hexahydrate (MgCl₂·6H₂O), sodium acetate trihydrate (CH₃COONa·3H₂O), and sodium caprylate,·
and wherein
sodium chloride (NaCl) is comprised in a concentration of between 2 and 7 g/l, e.g. 2, 3, 4, 5, 6, or 7 g/l,
potassium chloride (KCl) is comprised in a concentration of between 0.2 and 0.5 g/l, e.g. 0.2, 0.3, 0.4, or 0.5 g/l,
calcium chloride dihydrate (CaCl₂ ·2H₂O) is comprised in a concentration of between 0.2 and 0.5 g/l, e.g. 0.2, 0.3, 0.4, or 0.5 g/l,
magnesium chloride hexahydrate (MgCl₂·6H₂O) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa·3H₂O) is comprised in a concentration of between 3 and 7 g/l, e.g. 3, 4, 5, 6, or 7 g/l, and/or
sodium caprylate is comprised in a concentration of between 0.05 and 0.07 g/l, e.g. 0.05, 0.06, or 0.07 g/l.

In one even more preferred embodiment, the salt solution further comprises sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), and sodium caprylate.·

In one particularly even more preferred embodiment, the salt solution further comprises sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), and sodium caprylate,·
and wherein
sodium chloride (NaCl) is comprised in a concentration of between 2 and 7 g/l, e.g. 2, 3, 4, 5, 6, or 7 g/l,
potassium chloride (KCl) is comprised in a concentration of between 0.2 and 0.5 g/l, e.g. 0.2, 0.3, 0.4, or 0.5 g/l,
calcium chloride dihydrate (CaCl₂·2H₂O) is comprised in a concentration of between 0.2 and 0.5 g/l, e.g. 0.2, 0.3, 0.4, or 0.5 g/l,
magnesium chloride hexahydrate (MgCl₂·6H₂O) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa·3H₂O) is comprised in a concentration of between 3 and 7 g/l, e.g. 3, 4, 5, 6, or 7 g/l, and/or
sodium caprylate is comprised in a concentration of between 0.05 and 0.07 g/l, e.g. 0.05, 0.06, or 0.07 g/l.

Optionally, the salt solution further comprises N-acetyl-DL-tryptophan.

Particularly, the salt solution further comprises one or more salts selected from the group consisting of sodium chloride (NaCI), potassium chloride (KCl), calcium chloride dihydrate (CaCl_{2·}2H₂O), magnesium chloride hexahydrate (MgCl₂-6H₂O), sodium acetate trihydrate (CH₃COONa·3H₂O), sodium caprylate, and N-acetyl-DL-tryptophan.

More particularly, the salt solution further comprises sodium chloride (NaCI), potassium chloride (KCl), calcium chloride dihydrate (CaCl_{2·}2H₂O), magnesium chloride hexahydrate (MgCl₂-6H₂O), sodium acetate trihydrate (CH₃COONa·3H₂O), sodium caprylate, and N-acetyl-DL-tryptophan.

Preferably, N-acetyl-DL-tryptophan is comprised in a concentration of between 0.04 and 0.1 g/l, e.g. 0.04, 0.05, 0.06, 0.07, 0.08, 0.09, or 0.1 g/l.

More preferably, N-acetyl-DL-tryptophan is comprised in a concentration of between 0.05 and 0.09 g/l, e.g. 0.05, 0.06, 0.07, 0.08, or 0.09 g/l.

Even more preferably, N-acetyl-DL-tryptophan is comprised in a concentration of between 0.06 and 0.08 g/l, e.g. 0.06, 0.07, or 0.08 g/l.

Specifically, the salt solution further comprises one or more salts selected from the group consisting of sodium chloride (NaCI), potassium chloride (KCl), calcium chloride dihydrate (CaCl_{2·}2H₂O), magnesium chloride hexahydrate (MgCl₂-6H₂O), sodium acetate trihydrate (CH₃COONa·3H₂O), sodium caprylate, and N-acetyl-DL-tryptophan,
and wherein
sodium chloride (NaCl) is comprised in a concentration of between 2 and 7 g/l, e.g. 2, 3, 4, 5, 6, or 7 g/l,
potassium chloride (KCl) is comprised in a concentration of between 0.2 and 0.5 g/l, e.g. 0.2, 0.3, 0.4, or 0.5 g/l,
calcium chloride dihydrate (CaCl₂·2H₂O) is comprised in a concentration of between 0.2 and 0.5 g/l, e.g. 0.2, 0.3, 0.4, or 0.5 g/l,
magnesium chloride hexahydrate (MgCl₂·6H₂O) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa·3H₂O) is comprised in a concentration of between 3 and 7 g/l, e.g. 3, 4, 5, 6, or 7 g/l,
sodium caprylate·is comprised in a concentration of between 0.05 and 0.07 g/l, e.g. 0.05, 0.06, or 0.07 g/l, and/or
N-acetyl-DL-tryptophan is comprised in a concentration of between 0.04 and 0.1 g/l, e.g. 0.04, 0.05, 0.06, 0.07, 0.08, 0.09, or 0,1 g/l.

More specifically, the salt solution further comprises sodium chloride (NaCI), potassium chloride (KCl), calcium chloride dihydrate (CaCl_{2·}2H₂O), magnesium chloride hexahydrate (MgCl₂-6H₂O), sodium acetate trihydrate (CH₃COONa·3H₂O), sodium caprylate, and N-acetyl-DL-tryptophan,
and wherein
sodium chloride (NaCl) is comprised in a concentration of between 2 and 7 g/l, e.g. 2, 3, 4, 5, 6, or 7 g/l,
potassium chloride (KCl) is comprised in a concentration of between 0.2 and 0.5 g/l, e.g. 0.2, 0.3, 0.4, or 0.5 g/l,
calcium chloride dihydrate (CaCl₂·2H₂O) is comprised in a concentration of between 0.2 and 0.5 g/l, e.g. 0.2, 0.3, 0.4, or 0.5 g/l,
magnesium chloride hexahydrate (MgCl₂·6H₂O) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa·3H₂O) is comprised in a concentration of between 3 and 7 g/l, e.g. 3, 4, 5, 6, or 7 g/l,
sodium caprylate is comprised in a concentration of between 0.05 and 0.07 g/l, e.g. 0.05, 0.06, or 0.07 g/l, and/or
N-acetyl-DL-tryptophan is comprised in a concentration of between 0.04 and 0.1 g/l, e.g. 0.04, 0.05, 0.06, 0.07, 0.08, 0.09, or 0,1 g/l.

In one still even more preferred embodiment, the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water,
   and wherein
the salt solution further comprises one or more salts selected from the group consisting of sodium chloride (NaCI), potassium chloride (KCl), calcium chloride dihydrate (CaCl₂ ·2H₂O), magnesium chloride hexahydrate (MgCl₂-6H₂O), sodium acetate trihydrate (CH₃COONa·3H₂O), sodium caprylate, and N-acetyl-DL-tryptophan.

In one particularly still even more preferred embodiment,
serum albumin is comprised in a concentration of between 10 and 30 g/l, e.g. 10, 11, 12, 13, 14, 15, 16, 17, 18, 19, 20, 21, 22, 23, 24, 25, 26, 27, 28, 29, or 30 g/l,
alpha-1-acid glycoprotein (if present) is comprised in a concentration of between 0.005 and 0.4 g/l, e.g. 0.005, 0.006, 0.007, 0.008, 0.009, 0.01, 0.02, 0.03, 0.04, 0.05, 0.06, 0.07, 0.08, 0.09, 0.1, 0.2, 0.3, or 0.4 g/l,
immunoglobulin G (if present) is comprised in a concentration of between 1×10⁻⁷ and 4x10⁻⁴ g/l, deuterated water (if present) is comprised in a concentration of between 0.15 and 500 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, or 500 ml/l,
sodium chloride (NaCl) (if present) is comprised in a concentration of between 2 and 7 g/l, e.g. 2, 3, 4, 5, 6, or 7 g/l,
potassium chloride (KCl) (if present) is comprised in a concentration of between 0.2 and 0.5 g/l, e.g. 0.2, 0.3, 0.4, or 0.5 g/l,
calcium chloride dihydrate (CaCl₂·2H₂O) (if present) is comprised in a concentration of between 0.2 and 0.5 g/l, e.g. 0.2, 0.3, 0.4, or 0.5 g/l,
magnesium chloride hexahydrate (MgCl₂·6H₂O) (if present) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa-3H₂O) (if present) is comprised in a concentration of between 3 and 7 g/l, e.g. 3, 4, 5, 6, or 7 g/l,
sodium caprylate·(if present) is comprised in a concentration of between 0.05 and 0.07 g/l, e.g. 0.05, 0.06, or 0.07 g/l, and/or
N-acetyl-DL-tryptophan (if present) is comprised in a concentration of between 0.04 and 0.1 g/l, e.g. 0.04, 0.05, 0.06, 0.07, 0.08, 0.09, or 0,1 g/l.
The above-mentioned serum albumin is specifically human serum albumin.
Instead of deuterated water, urea can be used. Preferably, urea is comprised in a concentration of between 0.18 and 5 g/l, e.g. 0.18, 0.19, 0.2, 0.3, 0.4, 0.5, 0.6, 0.7, 0.8, 0.9, 1, 2, 3, 4, or 5 g/l. More preferably, urea is comprised in a concentration of between 0.3 and 2 g/l, e.g. 0.3, 0.4, 0.5, 0.6, 0.7, 0.8, 0.9, 1, or 2 g/l.

In one most preferred embodiment, the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water,
and wherein the salt solution further comprises sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), sodium caprylate and optionally N-acetyl-DL-tryptophan.·
In one particularly most preferred embodiment,
serum albumin is comprised in a concentration of between 10 and 30 g/l, e.g. 10, 11, 12, 13, 14, 15, 16, 17, 18, 19, 20, 21, 22, 23, 24, 25, 26, 27, 28, 29, or 30 g/1,
alpha-1-acid glycoprotein (if present) is comprised in a concentration of between 0.005 and 0.4 g/l, e.g. 0.005, 0.006, 0.007, 0.008, 0.009, 0.01, 0.02, 0.03, 0.04, 0.05, 0.06, 0.07, 0.08, 0.09, 0.1, 0.2, 0.3, or 0.4 g/l,
immunoglobulin G (if present) is comprised in a concentration of between 1×10⁻⁷ and 4x10⁻⁴ g/l, deuterated water (if present) is comprised in a concentration of between 0.15 and 500 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, or 500 ml/l,
sodium chloride (NaCl) is comprised in a concentration of between 2 and 7 g/l, e.g. 2, 3, 4, 5, 6, or 7 g/l,
potassium chloride (KCl) is comprised in a concentration of between 0.2 and 0.5 g/l, e.g. 0.2, 0.3, 0.4, or 0.5 g/l,
calcium chloride dihydrate (CaCl₂·2H₂O) is comprised in a concentration of between 0.2 and 0.5 g/l, e.g. 0.2, 0.3, 0.4, or 0.5 g/l,
magnesium chloride hexahydrate (MgCl₂·6H₂O) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa-3H₂O) is comprised in a concentration of between 3 and 7 g/l, e.g. 3, 4, 5, 6, or 7 g/l,
sodium caprylate is comprised in a concentration of between 0.05 and 0.07 g/l, e.g. 0.05, 0.06, or 0.07 g/l, and/or
optionally N-acetyl-DL-tryptophan is comprised in a concentration of between 0.04 and 0.1 g/l, e.g. 0.04, 0.05, 0.06, 0.07, 0.08, 0.09, or 0,1 g/l.
The above-mentioned serum albumin is specifically human serum albumin.
Instead of deuterated water, urea can be used. Preferably, urea is comprised in a concentration of between 0.18 and 5 g/l, e.g. 0.18, 0.19, 0.2, 0.3, 0.4, 0.5, 0.6, 0.7, 0.8, 0.9, 1, 2, 3, 4, or 5 g/l. More preferably, urea is comprised in a concentration of between 0.3 and 2 g/l, e.g. 0.3, 0.4, 0.5, 0.6, 0.7, 0.8, 0.9, 1, or 2 g/l.

In one specific example, the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water,
   and one or more salts selected from the group consisting of sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), sodium caprylate, and N-acetyl-DL-tryptophan, wherein
serum albumin is comprised in a concentration of between 10 and 30 g/l, e.g. 10, 11, 12, 13, 14, 15, 16, 17, 18, 19, 20, 21, 22, 23, 24, 25, 26, 27, 28, 29, or 30 g/l,
alpha-1-acid glycoprotein (if present) is comprised in a concentration of between 0.005 and 0.4 g/l, e.g. 0.005, 0.006, 0.007, 0.008, 0.009, 0.01, 0.02, 0.03, 0.04, 0.05, 0.06, 0.07, 0.08, 0.09, 0.1, 0.2, 0.3, or 0.4 g/l,
immunoglobulin G (if present) is comprised in a concentration of between 1×10⁻⁷ and 4x10⁻⁴ g/l, deuterated water (if present) is comprised in a concentration of between 0.15 and 500 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, or 500 ml/l,
sodium chloride (NaCl) (if present) is comprised in a concentration of between 2 and 7 g/l, e.g. 2, 3, 4, 5, 6, or 7 g/l,
potassium chloride (KCl) (if present) is comprised in a concentration of between 0.2 and 0.5 g/1, e.g. 0.2, 0.3, 0.4, or 0.5 g/l,
calcium chloride dihydrate (CaCl₂·2H₂O) (if present) is comprised in a concentration of between 0.2 and 0.5 g/l, e.g. 0.2, 0.3, 0.4, or 0.5 g/l,
magnesium chloride hexahydrate (MgCl₂·6H₂O) (if present) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa-3H₂O) (if present) is comprised in a concentration of between 3 and 7 g/l, e.g. 3, 4, 5, 6, or 7 g/l,
sodium caprylate. (if present) is comprised in a concentration of between 0.05 and 0.07 g/l, e.g. 0.05, 0.06, or 0.07 g/l, and/or
N-acetyl-DL-tryptophan (if present) is comprised in a concentration of between 0.04 and 0.1 g/l, e.g. 0.04, 0.05, 0.06, 0.07, 0.08, 0.09, or 0,1 g/l.
The above-mentioned serum albumin is specifically human serum albumin.
Instead of deuterated water, urea can be used. Preferably, urea is comprised in a concentration of between 0.18 and 5 g/l, e.g. 0.18, 0.19, 0.2, 0.3, 0.4, 0.5, 0.6, 0.7, 0.8, 0.9, 1, 2, 3, 4, or 5 g/l. More preferably, urea is comprised in a concentration of between 0.3 and 2 g/l, e.g. 0.3, 0.4, 0.5, 0.6, 0.7, 0.8, 0.9, 1, or 2 g/l.

In one another specific example, the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water,
   and sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), sodium caprylate and optionally N-acetyl-DL-tryptophan, wherein
serum albumin is comprised in a concentration of between 10 and 30 g/l, e.g. 10, 11, 12, 13, 14, 15, 16, 17, 18, 19, 20, 21, 22, 23, 24, 25, 26, 27, 28, 29, or 30 g/1,
alpha-1-acid glycoprotein (if present) is comprised in a concentration of between 0.005 and 0.4 g/l, e.g. 0.005, 0.006, 0.007, 0.008, 0.009, 0.01, 0.02, 0.03, 0.04, 0.05, 0.06, 0.07, 0.08, 0.09, 0.1, 0.2, 0.3, or 0.4 g/l,
immunoglobulin G (if present) is comprised in a concentration of between 1×10⁻⁷ and 4x10⁻⁴ g/l, deuterated water (if present) is comprised in a concentration of between 0.15 and 500 ml/l, e.g. 0.15, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, or 500 ml/l,
sodium chloride (NaCl) is comprised in a concentration of between 2 and 7 g/l, e.g. 2, 3, 4, 5, 6, or 7 g/l,
potassium chloride (KCl) is comprised in a concentration of between 0.2 and 0.5 g/l, e.g. 0.2, 0.3, 0.4, or 0.5 g/l,
calcium chloride dihydrate (CaCl₂·2H₂O) is comprised in a concentration of between 0.2 and 0.5 g/l, e.g. 0.2, 0.3, 0.4, or 0.5 g/l,
magnesium chloride hexahydrate (MgCl₂·6H₂O) is comprised in a concentration of between 0.15 and 0.35 g/l, e.g. 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, or 0.35 g/l,
sodium acetate trihydrate (CH₃COONa-3H₂O) is comprised in a concentration of between 3 and 7 g/l, e.g. 3, 4, 5, 6, or 7 g/l,
sodium caprylate is comprised in a concentration of between 0.05 and 0.07 g/l, e.g. 0.05, 0.06, or 0.07 g/l, and/or
optionally N-acetyl-DL-tryptophan is comprised in a concentration of between 0.04 and 0.1 g/l, e.g. 0.04, 0.05, 0.06, 0.07, 0.08, 0.09, or 0,1 g/l.
The above-mentioned serum albumin is specifically human serum albumin.
Instead of deuterated water, urea can be used. Preferably, urea is comprised in a concentration of between 0.18 and 5 g/l, e.g. 0.18, 0.19, 0.2, 0.3, 0.4, 0.5, 0.6, 0.7, 0.8, 0.9, 1, 2, 3, 4, or 5 g/l. More preferably, urea is comprised in a concentration of between 0.3 and 2 g/l, e.g. 0.3, 0.4, 0.5, 0.6, 0.7, 0.8, 0.9, 1, or 2 g/l.

In a second aspect, the present invention relates to the use of the physiological salt solution of the first aspect as
dialysis solution for microdialysis (MD), or
perfusion solution for Open Flow Microperfusion (OFM).
The use is specifically for cosmetic or pharmaceutical purposes. It may be an *in vitro* or *in vivo* use.

Preferably,
the dialysis solution is used for bioequivalence studies with microdialysis (MD), or
the perfusion solution is used for bioequivalence studies with Open Flow Microperfusion (OFM). In the bioequivalence studies, the bioequivalence between a test substance and a reference substance is particularly determined. In this respect, it is also referred to the fourth aspect of the present invention.
Alternatively, in the bioequivalence studies, the bioequivalence between a first treatment site of an individual treated with a test substance and a second treatment site of the individual treated with the (same) test substance is particularly determined. In this respect, it is also referred to the fifth aspect of the present invention.

More preferably,
the microdialysis (MD) is dermal microdialysis (dMD), or
the Open Flow Microperfusion (OFM) is dermal Open Flow Microperfusion (dOFM).

In a third aspect, the present invention relates to a kit, said kit comprises:
(i) a physiological salt solution of the first aspect, and
(ii) a dialysis or perfusion tube.

The kit can specifically be used for cosmetic or pharmaceutical purposes. The use may be an *in vitro* or *in vivo* use. Particularly, the kit can be used for microdialysis (MD) such as dermal microdialysis (dMD) or Open Flow Microperfusion (OFM) such as dermal Open Flow Microperfusion (dOFM).

Preferably, the kit is used for microdialysis (MD) such as dermal microdialysis (dMD) or Open Flow Microperfusion (OFM) such as dermal Open Flow Microperfusion (dOFM).
In this case, it is preferred that the kit further comprises
(iii) additional means for carrying out microdialysis (MD) or Open Flow Microperfusion (OFM).

Said means encompass, for example, a dialysis tube (having a lumen and a semi-permeable membrane or a perfusion tube (having a lumen and a permeable membrane). Said means further encompass, for example, additional components to carry out the sampling process such as high precision pumps, pump tubings, containers for the dialysis solution or perfusion solution, and/or sampling tubes for the dialysate or perfusate. Specifically, said additional means for carrying out MD, such as dMD, encompass a dialysis tube, high precision pumps, pump tubings, containers for the dialysis solution, and/or sampling tubes for the dialysate. Alternatively, said additional means for carrying out OFM, such as dOFM, encompass a perfusion tube, high precision pumps, pump tubings, containers for the perfusion solution, and/or sampling tubes for the perfusate.

In a fourth aspect, the present invention relates to a method for determining bioequivalence between a test substance and a reference substance comprising the steps of:
(i) providing at least one dialysate or perfusate (e.g. 1, 2, 3, 4, or 5 dialysate(s) or perfusate(s)) comprising a test substance obtained from an individual treated on its skin with the test substance,
(ii) providing at least one dialysate or perfusate (e.g. 1, 2, 3, 4, or 5 dialysate(s) or perfusate(s)) comprising a reference substance obtained from the (same) individual treated on its skin with the reference substance,
(iii) determining at least one pharmacokinetic parameter (e.g. 1, 2, 3, 4, or 5 pharmacokinetic parameter(s)) of the test substance in the at least one dialysate or perfusate (e.g. 1, 2, 3, 4, or 5 dialysate(s) or perfusate(s)) provided in (i) and determining (the same) at least one pharmacokinetic parameter (e.g. 1, 2, 3, 4, or 5 pharmacokinetic parameter(s)) of the reference substance in the at least one dialysate or perfusate (e.g. 1, 2, 3, 4, or 5 dialysate(s) or perfusate(s)) provided in (ii), and
(iv) comparing the at least one pharmacokinetic parameter (e.g. 1, 2, 3, 4, or 5 pharmacokinetic parameter(s)) of the test substance with the (same) at least one pharmacokinetic parameter (e.g. 1, 2, 3, 4, or 5 pharmacokinetic parameter(s)) of the reference substance,
   wherein an equivalence/a comparability between the at least one pharmacokinetic parameter of the test substance with the (same) at least one pharmacokinetic parameter of the reference substance indicates bioequivalence of the test substance and the reference substance.

It should be noted that the dialysate or perfusate is obtained from the individual treated on its skin with a test substance and a reference substance having the same dose, concentration, or amount. In addition, the treatment was carried out under similar conditions, specifically at comparable skin areas, especially at skin areas in the immediate vicinity. Moreover, important surrounding parameters like temperature and humidity were monitored/controlled. Thus, both substances were applied/used in the same way and under similar conditions. This allows comparability.

The dialysate may be obtained via dermal microdialysis (dMD). The dermal microdialysis (dMD) requires the insertion of a small dialysis catheter (also referred to as dialysis tube) into the skin. The dialysis tube is designed to mimic a capillary and consists of a shaft with a semipermeable hollow fiber membrane at its tip, which is connected to inlet and outlet tubing. The probe is continuously streamed with an aqueous solution (dialysis solution) that closely resembles the (ionic) composition of the surrounding tissue fluid at a low flow rate. This solution is preferably the physiological salt solution of the first aspect. Once inserted into the skin, small solutes can cross the semipermeable membrane by passive diffusion. The direction of the substance flow is determined by the respective concentration gradient and allows the usage of microdialysis probes as sampling as well as delivery tools. The solution leaving the probe (dialysate) is collected at certain time intervals for further analysis.

The perfusate may be obtained via Open Flow Microperfusion (dOFM). The dermal Open Flow Microperfusion (dOFM) requires the insertion of a small dialysis catheter (also referred to as perfusion tube) into the skin. The perfusion tube is designed to mimic a capillary and consists of a shaft with a permeable hollow fiber membrane at its tip, which is connected to inlet and outlet tubing. The probe is continuously streamed with an aqueous solution (perfusion solution) that closely resembles the (ionic) composition of the surrounding tissue fluid at a low flow rate. This solution is preferably the physiological salt solution of the first aspect. Once inserted into the skin, small solutes can cross the permeable membrane by passive diffusion. The direction of the substance flow is determined by the respective concentration gradient and allows the usage of Open Flow Microperfusion probes as sampling as well as delivery tools. The solution leaving the probe (perfusate) is collected at certain time intervals for further analysis.

Thus, in one embodiment
the dialysate is/has been obtained from the individual via a dialysis tube embedded/inserted into the tissue underlying the skin of said individual, or
the perfusate is/has been obtained from the individual via a perfusion tube embedded/inserted into the tissue underlying the skin of said individual.
Both, the dialysis or perfusion tube comprises a lumen. While the dialysis tube is semi-permeable/comprises a semi-permeable membrane, the perfusion tube is permeable/comprises a permeable membrane.
The test substance or reference substance penetrated the skin and entered the dermal interstitial fluid that surrounds cells and tissue underlying the skin.
While the dialysis tube allowed the diffusion of the test substance or reference substance from the dermal intestinal fluid into a dialysis solution comprised in its lumen, the perfusion tube allowed the perfusion of the test substance or reference substance from the dermal intestinal fluid into a perfusion solution comprised in its lumen.

The dialysis solution or perfusion solution is preferably the physiological salt solution of the first aspect.

Specifically,
the dialysate comprising a test substance or reference substance further comprises a dialysis solution, preferably the physiological salt solution of the first aspect, and dermal interstitial fluid, or
the perfusate comprising a test substance or reference substance further comprises a perfusion solution, preferably the physiological salt solution of the first aspect, and dermal interstitial fluid.

In one preferred embodiment
the dialysate comprising a test substance or reference substance is obtained at a first point in time and at one or more later point(s) in time and the at least one pharmacokinetic parameter (e.g. 1, 2, 3, 4, or 5 pharmacokinetic parameter(s)) of the test substance or reference substance is determined in said dialysate, or
the perfusate comprising a test substance or reference substance is obtained at a first point in time and at one or more later point(s) in time and the at least one pharmacokinetic parameter (e.g. 1, 2, 3, 4, or 5 pharmacokinetic parameter(s)) of the test substance or reference substance is determined in said perfusate.
Preferably,
the first point in time is the time point of treatment of the skin with the test substance or reference substance, and/or
the one or more later point(s) in time are from 4 to 48 hours, particularly from 4 to 36 hours, after the first time point of treatment of the skin with the test substance or reference substance.
For example, the one or more later point(s) in time are 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, and/or 48 hours after the first time point of treatment of the skin with the test substance or reference substance.
The time interval between the first point in time and the one or more later point(s) in time may be 1, 2, 3, 4, 5, 6, 7, or 8 hour(s).
More preferably, the time interval between the first point in time and the one or more later point(s) in time is 4 hours.
The time interval between the later points in time may be 1, 2, 3, 4, 5, 6, 7, or 8 hour(s).
Even more preferably, the time interval between the later points in time is 4 hours.
For example,
the first point in time is the time point of treatment of the skin with the test substance or reference substance,
the second point in time is 4 hours after the first time point,
the third point in time is 8 hours after the first time point,
the fourth point in time is 12 hours after the first time point,
the fifth point in time is 16 hours after the first time point,
the sixth point in time is 20 hours after the first time point, and
the seventh point in time is 24 hours after the first time point.

In one more preferred embodiment, a mean value from the pharmacokinetic parameters (e.g. 2, 3, 4, or 5 pharmacokinetic parameters) determined in said dialysate or perfusate at the first point in time and at the one or more later point(s) in time is calculated.

As mentioned above, the at least one pharmacokinetic parameter (e.g. 1, 2, 3, 4, or 5 pharmacokinetic parameter(s)) of the test substance is compared with the (same) at least one pharmacokinetic parameter (e.g. 1, 2, 3, 4, or 5 pharmacokinetic parameter(s)) of the reference substance in step (iv) of the method of the fourth aspect of the present invention,
wherein an equivalence/a comparability between the at least one pharmacokinetic parameter (e.g. 1, 2, 3, 4, or 5 pharmacokinetic parameter(s)) of the test substance with the (same) at least one pharmacokinetic parameter (e.g. 1, 2, 3, 4, or 5 pharmacokinetic parameter(s)) of the reference substance indicates bioequivalence of the test substance and the reference substance.

Thus, if the pharmacokinetic parameter(s) of the test substance and the reference substance are equivalent/comparable, the test substance and the reference substance are considered as bioequivalent.
When two products are said to be bioequivalent, they are expected to be, for all intents and purposes, the same.
Formulated in a different way, bioequivalence is the absence of a significant difference in the rate and extent to which the active ingredient or active moiety of the test substance and reference substance becomes available at the site of substance action when administered at the same molar dose under similar conditions in an appropriately designed study.
Specifically, the test substance and the reference substance are bioequivalent if they are pharmaceutically equivalent or pharmaceutical alternatives and their bioavailabilities, in terms of rate (Cmax and tmax) and extent of absorption (area under the curve), after administration of the same molar dose under the same conditions, are similar to such a degree that their effects can be expected to be essentially the same.
It is preferred that bioequivalence is defined as having been achieved if the 90% confidence interval (CI) of the mean maximum drug concentration (Cmax), and the area under the time-concentration curve (AUC) of the test substance (e.g. generic formulation of a drug) relative to a reference substance (e.g. reference brand-name drug) is within a range of about 70% to 135%, e.g. 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, or 135%.
It is more preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 75% to 130%, e.g. 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130%.
It is even more preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 80% to 125%, e.g. 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, or 125%.
Specifically, bioequivalence is met if the 90% confidence interval (CI) of the mean C max, AUC (0-t) and AUC (0-∞) of the test substance (e.g. generic formulation of a drug) relative to the reference substance (e.g. reference brand-name drug) is within 70% to 135%, more specifically within 75% to 130%, and even more specifically within 80% to 125%, e.g. 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, or 135%. Alternatively, an equivalent/a comparable pharmacokinetic parameter of the test substance and the reference substance preferably means in the context of the present invention that there is a variation of between 0 and 10%, e.g. 0, 0.1, 0.2, 0.3, 0.4, 0.5, 0.6, 0.7, 0.8, 0.9, 1, 2, 3, 4, 5, 6, 7, 8, 9, or 10%. Changes within this range are not substantially and, thus, not relevant. "Equivalent/comparable" in this respect may also mean that the detected pharmacokinetic parameter variation is within the accuracy of a measurement. The accuracy of a measurement depends on the measurement method used for determining the pharmacokinetic parameter. Preferably, the pharmacokinetic parameter is selected from the group consisting of absorption rate constant, bioavailability, apparent volume of distribution, steady-state volume of distribution, unbound fraction, rate of elimination, clearance, renal clearance, metabolic clearance, fraction excreted unchanged, elimination rate constant, biologic half-life, maximum concentration, area under the concentration vs. time curve (AUC) and toxicity, particularly dermal toxicity.

It is preferred that the bioequivalence determined meets the requirements set by the Food and Drug Administration (FDA).

It is further preferred that the individual is a mammal such as a human.

It is also preferred that the test or reference substance is an active pharmaceutical ingredient (API) or a dermal drug.

The method of the fourth aspect of the present invention is used to identify a test substance having an effective bioequivalence to the reference substance for administering to an individual.

Preferably, the test substance is a generic drug and the reference substance is a reference brand-name drug. More preferably, the generic drug has an effective bioequivalence to the reference brand-name drug for administering to an individual.

In a particularly preferred embodiment, the kit of the third aspect is used in the method of the fourth aspect.

In a fifth aspect, the present invention relates to a method for determining bioequivalence between a test substance and a reference substance using a kit of the third aspect. The kit for microdialysis (MD) or Open Flow Microperfusion (OFM) - of the third aspect - comprises:
(i) a physiological salt solution of the first aspect,
(ii) a dialysis or perfusion tube, and
(iii) optionally additional means for carrying out microdialysis (MD) or Open Flow Microperfusion (OFM).

As to preferred embodiments of the kit, it is referred to the third aspect.

Preferably, the method comprises the steps of:
(i) providing at least one dialysate or perfusate comprising a test substance obtained from an individual treated on its skin with the test substance,
(ii) providing at least one dialysate or perfusate comprising a reference substance obtained from the individual treated on its skin with the reference substance,
(iii) determining at least one pharmacokinetic parameter of the test substance in the at least one dialysate or perfusate provided in (i) and determining (the same) at least one pharmacokinetic parameter of the reference substance in the at least one dialysate or perfusate provided in (ii), and
(iv) comparing the at least one pharmacokinetic parameter of the test substance with the (same) at least one pharmacokinetic parameter of the reference substance,
   wherein an equivalence/a comparability between the at least one pharmacokinetic parameter of the test substance with the (same) at least one pharmacokinetic parameter of the reference substance indicates bioequivalence of the test substance and the reference substance.

Specifically,
the dialysate is/has been obtained from the individual via a dialysis tube embedded/inserted into the tissue underlying the skin of said individual, or
the perfusate is/has been obtained from the individual via a perfusion tube embedded/inserted into the tissue underlying the skin of said individual. The dialysis tube or perfusion tube is part of the kit.
Both, the dialysis or perfusion tube comprises a lumen.
The test substance or reference substance penetrated the skin and entered the dermal interstitial fluid that surrounds cells and tissue underlying the skin.
While the dialysis tube allowed the diffusion of the test substance or reference substance from the dermal intestinal fluid into a dialysis solution comprised in its lumen, the perfusion tube allowed the perfusion of the test substance or reference substance from the dermal intestinal fluid into a perfusion solution comprised in its lumen. The dialysis or perfusion solution is the physiological salt solution of the first aspect. This solution is also part of the kit.

More preferably,
the dialysate comprising a test substance or reference substance further comprises the physiological salt solution of the first aspect and dermal interstitial fluid, or
the perfusate comprising a test substance or reference substance further comprises the physiological salt solution of the first aspect and dermal interstitial fluid.

As to other preferred embodiments, with respect to this method, it is referred to the fourth aspect of the present invention.

In a sixth aspect, the present invention relates to a method for determining bioequivalence between a first treatment site of an individual treated with a test substance and a second treatment site of the individual treated with the (same) test substance comprising the steps of:
(i) providing at least one dialysate or perfusate (e.g. 1, 2, 3, 4, or 5 dialysate(s) or perfusate(s)) comprising a test substance obtained from an individual treated on its skin at a first treatment site with the test substance,
(ii) providing at least one dialysate or perfusate (e.g. 1, 2, 3, 4, or 5 dialysate(s) or perfusate(s)) comprising the (same) test substance obtained from the individual treated on its skin at a second treatment site with the (same) test substance,
(iii) determining at least one pharmacokinetic parameter (e.g. 1, 2, 3, 4, or 5 pharmacokinetic parameter(s)) of the test substance at the first treatment site in the at least one dialysate or perfusate (e.g. 1, 2, 3, 4, or 5 dialysate(s) or perfusate(s)) provided in (i) and determining (the same) at least one pharmacokinetic parameter (e.g. 1, 2, 3, 4, or 5 pharmacokinetic parameter(s)) of the (same) test substance at the second treatment site in the at least one dialysate or perfusate (e.g. 1, 2, 3, 4, or 5 dialysate(s) or perfusate(s)) provided in (ii), and
(iv) comparing the at least one pharmacokinetic parameter (e.g. 1, 2, 3, 4, or 5 pharmacokinetic parameter(s)) of the test substance at the first treatment site with the (same) at least one pharmacokinetic parameter (e.g. 1, 2, 3, 4, or 5 pharmacokinetic parameter(s)) of the (same) test substance at the second treatment site,
wherein an equivalence/a comparability between the at least one pharmacokinetic parameter (e.g. 1, 2, 3, 4, or 5 pharmacokinetic parameter(s)) of the test substance at the first treatment site with the (same) at least one pharmacokinetic parameter (e.g. 1, 2, 3, 4, or 5 pharmacokinetic parameter(s)) of the (same) test substance at the second treatment site indicates bioequivalence of the first treatment site of the individual treated with the test substance and the second treatment site of the individual treated with the (same) test substance.

The first treatment site and the second treatment site may be located at different or same body areas. Preferably, the first treatment site and the second treatment site are located at the same body area.

It should be noted that the treatment at the first treatment site and the treatment at the second treatment site were carried out under similar conditions. Thus, the test substance was used/applied at both treatment sites in the same way and/or at the same dose, concentration, or amount. In addition, important surrounding parameters like temperature and humidity were monitored/controlled. This allows comparability.

The dialysate may be obtained via dermal microdialysis (dMD). The dermal microdialysis (dMD) requires the insertion of a small dialysis catheter (also referred to as dialysis tube) into the skin. The dialysis tube is designed to mimic a capillary and consists of a shaft with a semipermeable hollow fiber membrane at its tip, which is connected to inlet and outlet tubing. The probe is continuously streamed with an aqueous solution (dialysis solution) that closely resembles the (ionic) composition of the surrounding tissue fluid at a low flow rate. This solution is preferably the physiological salt solution of the first aspect. Once inserted into the skin, small solutes can cross the semipermeable membrane by passive diffusion. The direction of the substance flow is determined by the respective concentration gradient and allows the usage of microdialysis probes as sampling as well as delivery tools. The solution leaving the probe (dialysate) is collected at certain time intervals for further analysis.

The perfusate may be obtained via Open Flow Microperfusion (dOFM). The dermal Open Flow Microperfusion (dOFM) requires the insertion of a small dialysis catheter (also referred to as perfusion tube) into the skin. The perfusion tube is designed to mimic a capillary and consists of a shaft with a permeable hollow fiber membrane at its tip, which is connected to inlet and outlet tubing. The probe is continuously streamed with an aqueous solution (perfusion solution) that closely resembles the (ionic) composition of the surrounding tissue fluid at a low flow rate. This solution is preferably the physiological salt solution of the first aspect. Once inserted into the skin, small solutes can cross the permeable membrane by passive diffusion. The direction of the substance flow is determined by the respective concentration gradient and allows the usage of Open Flow Microperfusion probes as sampling as well as delivery tools. The solution leaving the probe (perfusate) is collected at certain time intervals for further analysis.

Thus, in one embodiment
the dialysate is/has been obtained from the individual via a dialysis tube embedded/inserted into the tissue underlying the skin of said individual, or
the perfusate is/has been obtained from the individual via a perfusion tube embedded/inserted into the tissue underlying the skin of said individual.
Both, the dialysis or perfusion tube comprises a lumen. While the dialysis tube is semi-permeable/comprises a semi-permeable membrane, the perfusion tube is permeable/comprises a permeable membrane.
The test substance penetrated the skin (at the first treatment site and at the second treatment site) and entered the dermal interstitial fluid that surrounds cells and tissue underlying the skin (at the first treatment site and at the second treatment site).
While the dialysis tube allowed the diffusion of the test substance from the dermal intestinal fluid into a dialysis solution comprised in its lumen, the perfusion tube allowed the perfusion of the test substance from the dermal intestinal fluid into a perfusion solution comprised in its lumen.

The dialysis solution or perfusion solution is preferably the physiological salt solution of the first aspect.

Specifically,
the dialysate comprising a test substance further comprises a dialysis solution, preferably the physiological salt solution of the first aspect, and dermal interstitial fluid, or
the perfusate comprising a test substance further comprises a perfusion solution, preferably the physiological salt solution of the first aspect, and dermal interstitial fluid.

In one preferred embodiment
the dialysate comprising a test substance is obtained at a first point in time and at one or more later point(s) in time and the at least one pharmacokinetic parameter (e.g. 1, 2, 3, 4, or 5 pharmacokinetic parameter(s)) of the test substance is determined in said dialysate, or
the perfusate comprising a test substance is obtained at a first point in time and at one or more later point(s) in time and the at least one pharmacokinetic parameter (e.g. 1, 2, 3, 4, or 5 pharmacokinetic parameter(s)) of the test substance is determined in said perfusate.
Preferably,
the first point in time is the time point of treatment of the first treatment site or second treatment site with the test substance, and/or
the one or more later point(s) in time are from 4 to 48 hours, particularly from 4 to 36 hours, after the first time point of treatment of the first treatment site or second treatment site with the test substance.
For example, the one or more later point(s) in time are 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, and/or 48 hours after the first time point of treatment of the first treatment site or second treatment site with the test substance.
The time interval between the first point in time and the one or more later point(s) in time may be 1, 2, 3, 4, 5, 6, 7, or 8 hour(s).
More preferably, the time interval between the first point in time and the one or more later point(s) in time is 4 hours.
The time interval between the later points in time may be 1, 2, 3, 4, 5, 6, 7, or 8 hour(s).
Even more preferably, the time interval between the later points in time is 4 hours.
For example,
the first point in time is the time point of treatment of the first treatment site or second treatment site with the test substance,
the second point in time is 4 hours after the first time point,
the third point in time is 8 hours after the first time point,
the fourth point in time is 12 hours after the first time point,
the fifth point in time is 16 hours after the first time point,
the sixth point in time is 20 hours after the first time point, and
the seventh point in time is 24 hours after the first time point.

In one more preferred embodiment, a mean value from the pharmacokinetic parameters (e.g. 2, 3, 4, or 5 pharmacokinetic parameters) determined in said dialysate or perfusate at the first point in time and at the one or more later point(s) in time is calculated.

As mentioned above, the at least one pharmacokinetic parameter (e.g. 1, 2, 3, 4, or 5 pharmacokinetic parameter(s)) of the test substance at the first treatment site is compared with the (same) at least one pharmacokinetic parameter (e.g. 1, 2, 3, 4, or 5 pharmacokinetic parameter(s)) of the (same) test substance at the second treatment site,
wherein an equivalence/a comparability between the at least one pharmacokinetic parameter (e.g. 1, 2, 3, 4, or 5 pharmacokinetic parameter(s)) of the test substance at the first treatment site with the (same) at least one pharmacokinetic parameter (e.g. 1, 2, 3, 4, or 5 pharmacokinetic parameter(s)) of the (same) test substance at the second treatment site indicates bioequivalence of the first treatment site of the individual treated with the test substance and the second treatment site of the individual treated with the (same) test substance.

Thus, if the pharmacokinetic parameter(s) of the test substance at the first treatment site and the pharmacokinetic parameter(s) of the test substance at the second treatment site are equivalent/comparable, the treatment sites are considered as being bioequivalent.
Particularly, treatment sites are bioequivalent, if there is no significant difference in the rate and extent to which the active ingredient or active moiety of the test substance becomes available at the site of substance action when administered at the same molar dose under similar conditions in an appropriately designed study.
Specifically, the treatment sites are bioequivalent if the test substance at said treatment sites is pharmaceutically equivalent in terms of rate (Cmax and tmax) and extent of absorption (area under the curve) after administration of the same molar dose under the same conditions. The equivalence/similarity is to such a degree that the effect of the test substance at said treatment sites can be expected to be essentially the same.
It is preferred that bioequivalence is defined as having been achieved if the 90% confidence interval (CI) of the mean maximum drug concentration (Cmax), and the area under the time-concentration curve (AUC) of the test substance at the first treatment site relative to the (same) test substance at the second treatment site is within a range of about 70% to 135%, e.g. 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, or 135%.
It is more preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 75% to 130%, e.g. 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130%.
It is even more preferred that bioequivalence is defined as having been achieved if the 90% confidence interval (CI) of the mean maximum drug concentration (Cmax), and the area under the time-concentration curve (AUC) of the generic formulation of a drug (test substance) relative to a reference brand-name drug (reference substance) is within a range of about 80% to 125%, e.g. 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, or 125%.
Specifically, bioequivalence is met if the 90% confidence interval (CI) of the mean C max, AUC (0-t) and AUC (0-∞) of the test substance at the first treatment site relative to the (same) test substance at the second treatment site is within 70% to 135%, more specifically within 75% to 130%, even more specifically within 80% to 125%, e.g. 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, or 135%.
Alternatively, an equivalent/a comparable pharmacokinetic parameter of the test substance at the first treatment site and second treatment site means in the context of the present invention that there is a variation of between 0 and 10%, e.g. 0, 0.1, 0.2, 0.3, 0.4, 0.5, 0.6, 0.7, 0.8, 0.9, 1, 2, 3, 4, 5, 6, 7, 8, 9, or 10%. Changes within this range are not substantially and, thus, not relevant. "Equivalent/comparable" in this respect may also mean that the detected pharmacokinetic parameter variation is within the accuracy of a measurement. The accuracy of a measurement depends on the measurement method used for determining the pharmacokinetic parameter. Preferably, the pharmacokinetic parameter is selected from the group consisting of absorption rate constant, bioavailability, apparent volume of distribution, steady-state volume of distribution, unbound fraction, rate of elimination, clearance, renal clearance, metabolic clearance, fraction excreted unchanged, elimination rate constant, biologic half-life, maximum concentration, area under the concentration vs. time curve (AUC) and toxicity, particularly dermal toxicity.

It is preferred that the bioequivalence determined meets the requirements set by the Food and Drug Administration (FDA).

It is further preferred that the individual is a mammal such as a human.

It is also preferred that the test or reference substance is an active pharmaceutical ingredient (API) or a dermal drug.

In a particularly preferred embodiment, the kit of the third aspect is used in the method of the sixth aspect.

In a seventh aspect, the present invention relates to a method for determining bioequivalence between a first treatment site of an individual treated with a test substance and a second treatment site of the individual treated with the (same) test substance using a kit of the third aspect. The kit for microdialysis (MD) or Open Flow Microperfusion (OFM) - of the third aspect
- comprises:
   (i) a physiological salt solution of the first aspect,
   (ii) a dialysis or perfusion tube, and
   (iii) optionally additional means for carrying out microdialysis (MD) or Open Flow Microperfusion (OFM).
      As to preferred embodiments of the kit, it is referred to the third aspect.
      Preferably, the method comprises the steps of:
      (i) providing at least one dialysate or perfusate comprising a test substance obtained from an individual treated on its skin at a first treatment site with the test substance,
      (ii) providing at least one dialysate or perfusate comprising the (same) test substance obtained from the individual treated on its skin at a second treatment site with the (same) test substance,
      (iii) determining at least one pharmacokinetic parameter of the test substance at the first treatment site in the at least one dialysate or perfusate provided in (i) and determining (the same) at least one pharmacokinetic parameter of the (same) test substance at the second treatment site in the at least one dialysate or perfusate provided in (ii), and
      (iv) comparing the at least one pharmacokinetic parameter of the test substance at the first treatment site with the (same) at least one pharmacokinetic parameter of the (same) test substance at the second treatment site,
         wherein an equivalence/a comparability between the at least one pharmacokinetic parameter of the test substance at the first treatment site with the (same) at least one pharmacokinetic parameter of the (same) test substance at the second treatment site indicates bioequivalence of the first treatment site of the individual treated with the test substance and the second treatment site of the individual treated with the (same) test substance.

Specifically,
the dialysate is/has been obtained from the individual via a dialysis tube embedded/inserted into the tissue underlying the skin of said individual, or
the perfusate is/has been obtained from the individual via a perfusion tube embedded/inserted into the tissue underlying the skin of said individual. The dialysis tube or perfusion tube is part of the kit.
Both, the dialysis or perfusion tube comprises a lumen.
The test substance penetrated the skin (at the first treatment site and at the second treatment site) and entered the dermal interstitial fluid that surrounds cells and tissue underlying the skin (at the first treatment site and at the second treatment site).
While the dialysis tube allowed the diffusion of the test substance from the dermal intestinal fluid into a dialysis solution comprised in its lumen, the perfusion tube allowed the perfusion of the test substance from the dermal intestinal fluid into a perfusion solution comprised in its lumen. The dialysis or perfusion solution is the physiological salt solution of the first aspect. This solution is also part of the kit.

More preferably,
the dialysate comprising a test substance further comprises the physiological salt solution of the first aspect and dermal interstitial fluid, or
the perfusate comprising a test substance further comprises the physiological salt solution of the first aspect and dermal interstitial fluid.

As to other preferred embodiments, with respect to this method, it is referred to the sixth aspect of the present invention.

The invention is summarized as follows:
1. A physiological salt solution comprising serum albumin.
2. The physiological salt solution of item 1, wherein the serum albumin is comprised in a concentration of between 10 and 30 g/l.
3. The physiological salt solution of items 1 or 2, wherein the salt solution further comprises alpha-1-acid glycoprotein.
4. The physiological salt solution of item 3, wherein alpha-1-acid glycoprotein is comprised in a concentration of between 0.005 and 0.4 g/l.
5. The physiological salt solution of any one of items 1 to 4, wherein the salt solution further comprises immunoglobulin G.
6. The physiological salt solution of item 5, wherein immunoglobulin G is comprised in a concentration of between 1×10⁻⁷ and 4x10⁻⁴ g/l.
7. The physiological salt solution of any one of items 1 to 6, wherein the salt solution further comprises deuterated water (as solvent).
8. The physiological salt solution of item 7, wherein deuterated water is comprised in a concentration of between 0.15 and 500 ml/l.
9. The physiological salt solution of any one of items 1 to 8, wherein the salt solution comprises
   serum albumin and alpha-1-acid glycoprotein,
   serum albumin and immunoglobulin G,
   serum albumin, alpha-1-acid glycoprotein, and immunoglobulin G,
   serum albumin and deuterated water,
   serum albumin, alpha-1-acid glycoprotein, and deuterated water,
   serum albumin, immunoglobulin G, and deuterated water, or
   serum albumin, alpha-1-acid glycoprotein, immunoglobulin G, and deuterated water.
10. The physiological salt solution of item 9, wherein
   serum albumin is comprised in a concentration of between 10 and 30 g/l,
   alpha-1-acid glycoprotein is comprised in a concentration of between 0.005 and 0.4 g/l,
   immunoglobulin G is comprised in a concentration of between 1×10⁻⁷ and 4x10⁻⁴ g/l, and/or
   deuterated water is comprised in a concentration of between 0.15 and 500 ml/l.
11. The physiological salt solution of any one of items 1 to 10, wherein the serum albumin is human serum albumin.
12. The physiological salt solution of any one of items 1 to 11, wherein the salt solution further comprises one or more salts selected from the group consisting of sodium chloride (NaCI), potassium chloride (KCl), calcium chloride dihydrate (CaCl₂·2H₂O), magnesium chloride hexahydrate (MgCl₂-6H₂O), sodium acetate trihydrate (CH₃COONa·3H₂O), and sodium caprylate.·
13. The physiological salt solution of item 12, wherein the salt solution further comprises sodium chloride (NaCI), potassium chloride (KCl), calcium chloride dihydrate (CaCl₂·2H₂O), magnesium chloride hexahydrate (MgCl₂-6H₂O), sodium acetate trihydrate (CH₃COONa·3H₂O), and sodium caprylate.
14. The physiological salt solution of items 12 or 13, wherein
   sodium chloride (NaCl) is comprised in a concentration of between 2 and 7 g/l,
   potassium chloride (KCl) is comprised in a concentration of between 0.2 and 0.5 g/l,
   calcium chloride dihydrate (CaCl₂·2H₂O) is comprised in a concentration of between 0.2 and 0.5 g/l,
   magnesium chloride hexahydrate (MgCl₂·6H₂O) is comprised in a concentration of between 0.15 and 0.35 g/l,
   sodium acetate trihydrate (CH₃COONa-3H₂O) is comprised in a concentration of between 3 and 7 g/l, and/or
   sodium caprylate·is comprised in a concentration of between 0.05 and 0.07 g/l.
15. The physiological salt solution of any one of items 1 to 14, wherein the salt solution further comprises N-acetyl-DL-tryptophan.
16. The physiological salt solution of item 15, wherein N-acetyl-DL-tryptophan is comprised in a concentration of between 0.04 and 0.1 g/l.
17. The physiological salt solution of item 16, wherein
   sodium chloride (NaCl) is comprised in a concentration of between 2 and 7 g/l,
   potassium chloride (KCl) is comprised in a concentration of between 0.2 and 0.5 g/l,
   calcium chloride dihydrate (CaCl₂·2H₂O) is comprised in a concentration of between 0.2 and 0.5 g/l,
   magnesium chloride hexahydrate (MgCl₂·6H₂O) is comprised in a concentration of between 0.15 and 0.35 g/l,
   sodium acetate trihydrate (CH₃COONa-3H₂O) is comprised in a concentration of between 3 and 7 g/l,
   sodium caprylate·is comprised in a concentration of between 0.03 and 0.07 g/l, and N-acetyl-DL-tryptophan is comprised in a concentration of between 0.04 and 0.1 g/l.
18. The physiological salt solution of any one of items 9 to 17, wherein the salt solution comprises
   serum albumin and alpha-1-acid glycoprotein,
   serum albumin and immunoglobulin G,
   serum albumin, alpha-1-acid glycoprotein, and immunoglobulin G,
   serum albumin and deuterated water,
   serum albumin, alpha-1-acid glycoprotein, and deuterated water,
   serum albumin, immunoglobulin G, and deuterated water, or
   serum albumin, alpha-1-acid glycoprotein, immunoglobulin G, and deuterated water, and wherein the salt solution further comprises
   sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), sodium caprylate and optionally N-acetyl-DL-tryptophan.·
19. The physiological salt solution of item 18, wherein serum albumin, preferably human serum albumin, is comprised in a concentration of between 10 and 30 g/l,
   the alpha-1-acid glycoprotein is comprised in a concentration of between 0.005 and 0.4 g/l,
   immunoglobulin G is comprised in a concentration of between 1×10⁻⁷ and 4×10⁻⁴ g/l,
   deuterated water is comprised in a concentration of between 0.15 and 500 ml/l,
   sodium chloride (NaCl) is comprised in a concentration of between 2 and 7 g/l,
   potassium chloride (KCl) is comprised in a concentration of between 0.2 and 0.5 g/l,
   calcium chloride dihydrate (CaCl₂·2H₂O) is comprised in a concentration of between 0.2 and 0.5 g/l,
   magnesium chloride hexahydrate (MgCl₂·6H₂O) is comprised in a concentration of between 0.15 and 0.35 g/l,
   sodium acetate trihydrate (CH₃COONa-3H₂O) is comprised in a concentration of between 3 and 7 g/l,
   sodium caprylate·is comprised in a concentration of between 0.03 and 0.07 g/l, and/or N-acetyl-DL-tryptophan is comprised in a concentration of between 0.04 and 0.1 g/l.
20. The physiological salt solution of any one of items 1 to 19, wherein the solution has a pH of between 6.0 and 7.5.
21. The physiological salt solution of any one of items 1 to 20, wherein the solution has an osmolarity of between 300 and 310 mOsm/l, preferably of 302 mOsm/l.
22. Use of the physiological salt solution of any one of items 1 to 21 as
   dialysis solution for microdialysis (MD), or
   perfusion solution for Open Flow Microperfusion (OFM).
23. The use of item 22, wherein
   the dialysis solution is used for bioequivalence studies with microdialysis (MD), or
   the perfusion solution is used for bioequivalence studies with Open Flow Microperfusion (OFM).
24. The use of item 23, wherein the bioequivalence between a test substance and a reference substance is determined.
25. The use of item 23, wherein the bioequivalence between a first treatment site of an individual treated with a test substance and a second treatment site of the individual treated with the (same) test substance is determined.
26. The use of any one of items 22 to 25, wherein
   the microdialysis (MD) is dermal microdialysis (dMD), or
   the Open Flow Microperfusion (OFM) is dermal Open Flow Microperfusion (dOFM).
27. A kit for microdialysis (MD) or Open Flow Microperfusion (OFM), said kit comprises:
   (i) a physiological salt solution of any one of items 1 to 21, and
   (ii) a dialysis or perfusion tube.
28. The kit of item 27, wherein the kit further comprises
   (iii) additional means for carrying out microdialysis (MD) or Open Flow Microperfusion (OFM).
29. A method for determining bioequivalence between a test substance and a reference substance comprising the steps of:
   (i) providing at least one dialysate or perfusate comprising a test substance obtained from an individual treated on its skin with the test substance,
   (ii) providing at least one dialysate or perfusate comprising a reference substance obtained from the individual treated on its skin with the reference substance,
   (iii) determining at least one pharmacokinetic parameter of the test substance in the at least one dialysate or perfusate provided in (i) and determining (the same) at least one pharmacokinetic parameter of the reference substance in the at least one dialysate or perfusate provided in (ii), and
   (iv) comparing the at least one pharmacokinetic parameter of the test substance with the (same) at least one pharmacokinetic parameter of the reference substance, wherein an equivalence/a comparability between the at least one pharmacokinetic parameter of the test substance with the (same) at least one pharmacokinetic parameter of the reference substance indicates bioequivalence of the test substance and the reference substance.
30. The method of item 29, wherein
   the dialysate is obtained from the individual via a dialysis tube embedded/inserted into the tissue underlying the skin of said individual, or
   the perfusate is obtained from the individual via a perfusion tube embedded/inserted into the tissue underlying the skin of said individual.
31. The method of item 30, wherein the dialysis or perfusion tube comprises a lumen.
32. The method of any one of items 29 to 31, wherein the test substance or reference substance penetrated the skin and entered the dermal interstitial fluid that surrounds cells and tissue underlying the skin.
33. The method of any one of items 30 to 32, wherein
   the dialysis tube allowed the diffusion of the test substance or reference substance from the dermal intestinal fluid into a dialysis solution comprised in its lumen, or
   the perfusion tube allowed the perfusion of the test substance or reference substance from the dermal intestinal fluid into a perfusion solution comprised in its lumen.
34. The method of item 33, wherein the dialysis solution or perfusion solution is a physiological salt solution of any one of items 1 to 21.
35. The method of any one of items 29 to 34, wherein
   the dialysate comprising a test substance or reference substance is obtained at a first point in time and at one or more later point(s) in time and the at least one pharmacokinetic parameter of the test substance or reference substance is determined in said dialysate, or the perfusate comprising a test substance or reference substance is obtained at a first point in time and at one or more later point(s) in time and the at least one pharmacokinetic parameter of the test substance or reference substance is determined in said perfusate.
36. The method of item 35, wherein the first point in time is the time point of treatment of the skin with the test substance or reference substance.
37. The method of items 35 or 36, wherein the one or more later point(s) in time are from 4 to 36 hours after the first time point of treatment of the skin with the test substance or reference substance.
38. The method of any one of items 35 to 37, wherein the time interval between the later points in time is 4 hours.
39. The method of any one of items 35 to 38, wherein a mean value from the pharmacokinetic parameters determined in said dialysate or perfusate at the first point in time and at the one or more later point(s) in time is calculated.
40. The method of any one of items 29 to 39, wherein the pharmacokinetic parameter is selected from the group consisting of absorption rate constant, bioavailability, apparent volume of distribution, steady-state volume of distribution, unbound fraction, rate of elimination, clearance, renal clearance, metabolic clearance, fraction excreted unchanged, elimination rate constant, biologic half-life, maximum concentration, area under the concentration vs. time curve (AUC) and toxicity, preferably dermal toxicity.
41. The method of any one of items 29 to 40, wherein bioequivalence is met if the 90% confidence interval (CI) of the mean C max, AUC (0-t) and AUC (0-∞) of the test substance relative to the reference substance is within 80% to 125%.
42. The method of any one of items 29 to 41, wherein the bioequivalence determined meets the requirements set by the Food and Drug Administration (FDA).
43. The method of any one of items 29 to 42, wherein the individual is a mammal.
44. The method of item 43, wherein the individual is a human.
45. The method of any one of items 29 to 44, wherein the test or reference substance is an active pharmaceutical ingredient (API) or a dermal drug.
46. The method of any one of items 29 to 45, wherein the method is used to identify a test substance having an effective bioequivalence to the reference substance for administering to an individual.
47. The method of item 46, wherein the test substance is a generic drug and the reference substance is a reference brand-name drug.
48. The method of item 47, wherein the generic drug has an effective bioequivalence to the reference brand-name drug for administering to an individual.
49. The method of any one of items 29 to 48, wherein
   the dialysate comprising a test substance or reference substance further comprises a dialysis solution, preferably a physiological salt solution of any one of items 1 to 21, and dermal interstitial fluid, or
   the perfusate comprising a test substance or reference substance further comprises a perfusion solution, preferably a physiological salt solution of any one of items 1 to 21, and dermal interstitial fluid.
50. A method for determining bioequivalence between a first treatment site of an individual treated with a test substance and a second treatment site of the individual treated with the (same) test substance comprising the steps of:
   (i) providing at least one dialysate or perfusate comprising a test substance obtained from an individual treated on its skin at a first treatment site with the test substance,
   (ii) providing at least one dialysate or perfusate comprising the (same) test substance obtained from the individual treated on its skin at a second treatment site with the (same) test substance,
   (iii) determining at least one pharmacokinetic parameter of the test substance at the first treatment site in the at least one dialysate or perfusate provided in (i) and determining (the same) at least one pharmacokinetic parameter of the (same) test substance at the second treatment site in the at least one dialysate or perfusate provided in (ii), and
   (iv) comparing the at least one pharmacokinetic parameter of the test substance at the first treatment site with the (same) at least one pharmacokinetic parameter of the (same) test substance at the second treatment site,
      wherein an equivalence/a comparability between the at least one pharmacokinetic parameter of the test substance at the first treatment site with the (same) at least one pharmacokinetic parameter of the (same) test substance at the second treatment site indicates bioequivalence of the first treatment site of the individual treated with the test substance and the second treatment site of the individual treated with the (same) test substance.
51. The method of item 50, wherein
   the dialysate is obtained from the individual via a dialysis tube embedded/inserted into the tissue underlying the skin of said individual, or
   the perfusate is obtained from the individual via a perfusion tube embedded/inserted into the tissue underlying the skin of said individual.
52. The method of item 51, wherein the dialysis or perfusion tube comprises a lumen.
53. The method of any one of items 50 to 52, wherein the test substance penetrated the skin and entered the dermal interstitial fluid that surrounds cells and tissue underlying the skin.
54. The method of any one of items 51 to 53, wherein
   the dialysis tube allowed the diffusion of the test substance from the dermal intestinal fluid into the dialysis solution comprised in its lumen, or
   the perfusion tube allowed the perfusion of the test substance from the dermal intestinal fluid into the perfusion solution comprised in its lumen.
55. The method of item 54, wherein the dialysis solution or perfusion solution is a physiological salt solution of any one of items 1 to 21.
56. The method of any one of items 50 to 55, wherein
   the dialysate comprising a test substance is obtained at a first point in time and at one or more later point(s) in time and the at least one pharmacokinetic parameter of the test substance is determined in said dialysate, or
   the perfusate comprising a test substance is obtained at a first point in time and at one or more later point(s) in time and the at least one pharmacokinetic parameter of the test substance is determined in said perfusate.
57. The method of item 56, wherein the first point in time is the time point of treatment of the first treatment site or second treatment site with the test substance.
58. The method of items 56 or 57, wherein the one or more later point(s) in time are from 4 to 36 hours after the first time point of treatment of the first treatment site or second treatment site with the test substance.
59. The method of any one of items 56 to 58, wherein the time interval between the later points in time is 4 hours.
60. The method of any one of items 56 to 59, wherein a mean value from the pharmacokinetic parameters determined in said dialysate or perfusate at the first point in time and at the one or more later point(s) in time is calculated.
61. The method of any one of items 50 to 60, wherein the pharmacokinetic parameter is selected from the group consisting of absorption rate constant, bioavailability, apparent volume of distribution, steady-state volume of distribution, unbound fraction, rate of elimination, clearance, renal clearance, metabolic clearance, fraction excreted unchanged, elimination rate constant, biologic half-life, maximum concentration, area under the concentration vs. time curve (AUC) and toxicity, preferably dermal toxicity.
62. The method of any one of items 50 to 61, wherein bioequivalence is met if the 90% confidence interval (CI) of the mean C max, AUC (0-t) and AUC (0-∞) of the test substance at the first treatment site relative to test substance at the second treatment site is within 80% to 125%.
63. The method of any one of items 50 to 62, wherein the bioequivalence determined meets the requirements set by the Food and Drug Administration (FDA).
64. The method of any one of items 50 to 63, wherein the individual is a mammal.
65. The method of item 64, wherein the individual is a human.
66. The method of any one of items 50 to 65 wherein
   the dialysate comprising a test substance further comprises a dialysis solution, preferably a physiological salt solution of any one of items 1 to 21, and dermal interstitial fluid, or the perfusate comprising a test substance further comprises a perfusion solution, preferably a physiological salt solution of any one of items 1 to 21, and dermal interstitial fluid.
67. The method of any one of items 50 to 66, wherein the first treatment site and the second treatment site are located at different or same body areas.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The present invention is further illustrated by the following Figures, however, without being restricted thereto.
**Figure 1****:** Shows a schema of dermal open flow dermal microperfusion (dOFM). The dOFM can assess the intradermal biochemistry and drug concentrations by sampling the perfusate for up to 48 h.
**Figure 2****:** Shows a schema (a) and photograph (b) of the duplicate test triad for comparative bioavailability assessment. Two treatment sites per test triad were dosed with the reference product (R₁: central, R₂: non-central) and one treatment site was dosed with the test product (T).
**Figure 3**: Shows in 3A concentration profiles in samples extracted with different sampling techniques and perfusates. Shows in 3B substance concentration (AUC) in samples extracted with different sampling techniques and perfusates.

### EXAMPLES

The present invention is further illustrated by the following examples, however, without being restricted thereto.

### 1. Development of the proprietary perfusion/dialysis solution

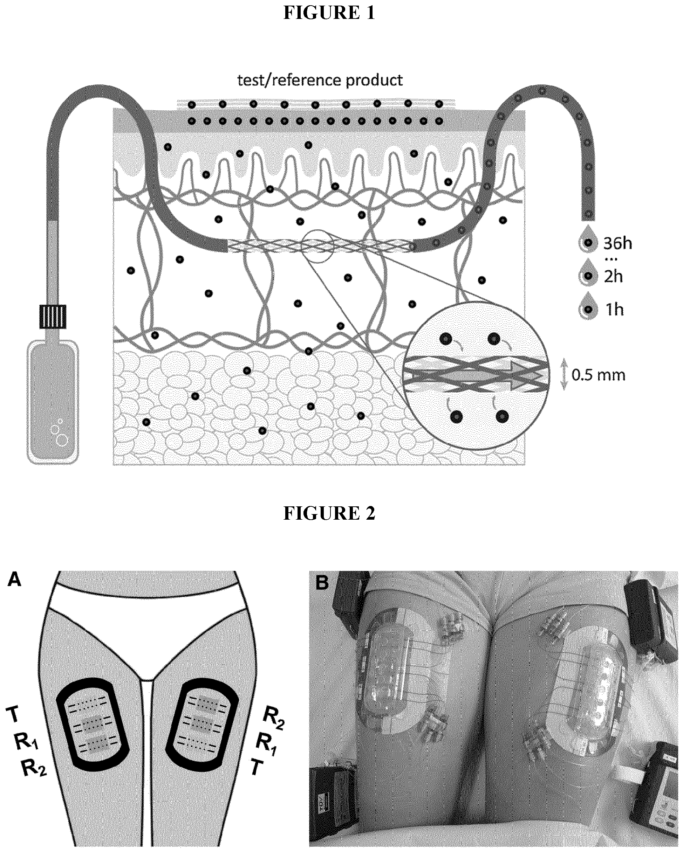

Recently, dermal open-flow microperfusion (dOFM) has been identified as a potential approach to assess the BA/BE of topical products. It facilitates a continuous assessment of the *in vivo* cutaneous kinetics of topically administered drugs directly in the dermis in human subjects. dOFM can assess the intradermal drug concentrations by sampling the dermal interstitial fluid for up to 48 h (**Figure 1**). The dOFM approach allows a direct PK approach for BE assessment in the dermis.

The study setup presented in **Figure 2** allows a direct comparison of two different topical drugs in the same healthy subject and avoids therefore high intersubject variability. It is possible to perform the same setup with microdialysis (MD).

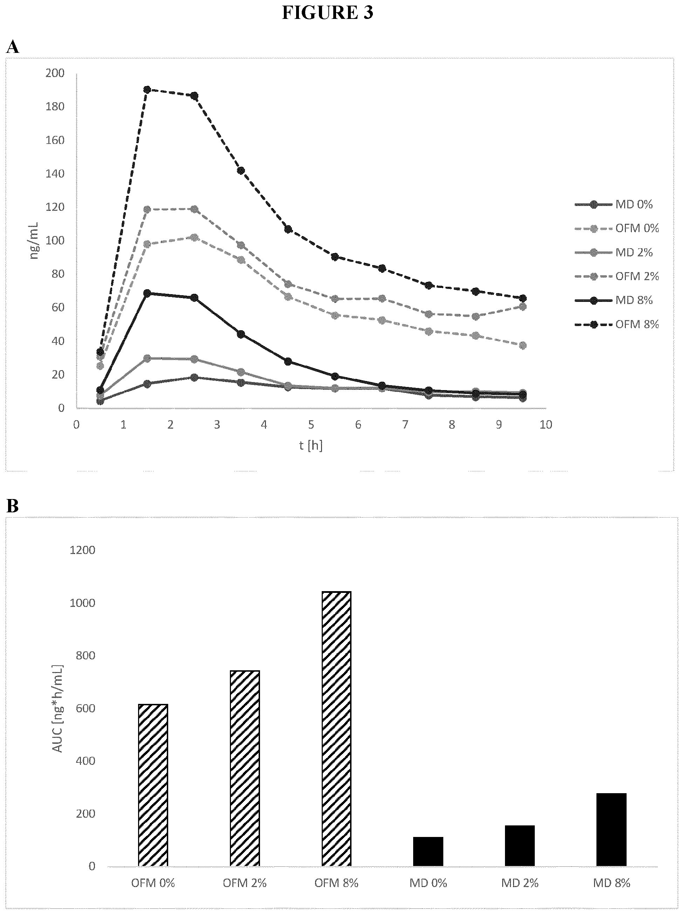

Continuous sampling systems like dOFM and MD rely on a liquid medium that is circulated through the probes and acts as a transport medium, the perfusion solution or dialysis solution. The composition of these solutions influences the degree of extraction of the substance that is being examined. The basic physical principals behind this effect are ionic strength, pH-value, osmotic, colloid osmotic and protein binding effects. The effects of an altering protein concentration in the perfusate is shown in **Figure 3**. The ideal perfusion solution or dialysis solution matches the dermal interstitial fluid in order to avoid complex exchange processes at the exchange area of dOFM and MD.

The present inventions have developed the following proprietary physiological salt solution which can be used with dOFM and MD set ups in order to avoid distortion of the results due to varying or inadequate compositions of the perfusion solution or dialysis solution:

**Table 1: Composition of the proprietary perfusion/dialysis solution for dermal BE studies with OFM and MD sampling**

| **Content** | **Formula** | **Concentration (g/1000 ml)** |
|---|---|---|
| **Sodium chloride** | **NaCl** | **5,45** |
| Potassium chloride | KCl | 0,34 |
| Calcium chloride dihydrate | CaCl₂ · 2H₂O | 0,33 |
| Magnesium chloride hexahydrate | MgCl₂ · 6H₂O | 0,27 |
| Sodium acetate trihydrate | CH₃COONa· 3H₂O | 5,51 |
| Human serum albumin | | 20,00 |
| Sodium caprylate | | 0,06 |
| N-acetyl-D,L-tryptophan | | 0,07 |
| Water for injection | | rest |
| Osmolarity: 302 mosmmol/l | | |
| pH value: 6.0-7.5 | | |

### 2. Determination of bioequivalence between a test substance and a reference substance as well as determination of bioequivalence between a first treatment site of an individual treated with a test substance and a second treatment site of the individual treated with the (same) test substance

The availability of generic topical dermatological drug products is constrained by the limited methods established to assess topical bioequivalence (BE). The novel cutaneous pharmacokinetic (PK) approaches, dermal open-flow microperfusion (dOFM) and dermal microdialysis (dMD), can continuously assess the rate and extent to which a topical drug becomes available in the dermis, to compare *in vivo* dermal bioavailability (BA) and support bioequivalence (BE) evaluations for topical products.

The aim of this experiment was to evaluate whether dOFM is an accurate, sensitive and reproducible *in vivo* method to characterize the BE and BA of acyclovir from 5% acyclovir creams, comparing a reference (R) substance either to itself or to a different test (T) substance.

In addition, the proprietary perfusion or dialysis solution as described herein was tested. It comes the composition of dermal interstitial fluid very close and, thus, is able to avoid complex exchange processes at the exchange area of dOFM and dMD. This improves dOFM and dMD

### 2.1 Study drugs

In this experiment, the BE and BA between a 5% acyclovir cream (Aciclovir 1A Pharma-Creme; 1A Pharma GmbH) as test substance (T) and another 5% acyclovir cream (Zovirax; Valeant, Bridgewater, NJ, USA) as reference substance (R) was determined. The experiment was conducted on healthy study subjects (test subjects).

### 2.2 Study solution

In this experiment, the physiological salt solution as described herein (see, for example, section 1) was used.

### 2.3 Experimental set-up

In a single-center clinical study, the R or T products were applied to six randomized treatment sites on the skin of 20 healthy human subjects (test subjects). Two dOFM probes were inserted in each treatment site to monitor the intradermal acyclovir concentration for 36 h. Comparative bioavailability (BA) (of R₂ versus R₁ and T versus R₁) was evaluated based on conventional bioequivalence (BE) criteria for PK endpoints (AUC and Cₘₐₓ), where the 90% confidence interval of the geometric mean ratio between the T and R fall within 0.80-1.25. In particular, BE was confirmed for the positive control products (R₂ versus R₁). In addition, negative control products (T versus R1) failed to demonstrate BE. The experimental set-up is further shown in **Figures 1** and **2**.

### 2.4 Determination bioequivalence (BE)

The positive control products (R₂ versus R₁) were accurately and reproducibly confirmed to be bioequivalent, while the negative control products (T versus R₁) were sensitively discriminated not to be bioequivalent. Thus, dOFM accurately, sensitively and reproducibly characterized the dermal BA in a manner that can support BE evaluations for topical acyclovir 5% creams in a study with n=40 (20 subjects in this study).

Similar results were obtained for dermal microdialysis (dMD) using the physiological salt solution as described herein (see, for example, section 1) (data not shown).

### 2.5 Summary

The present inventors could show the utility of clinical dOFM as a dermal PK approach to compare dermal BA and support BE evaluations for a topical (locally acting) drug product. The present inventors could further show that dOFM is capable of directly measuring the penetration of topically applied acyclovir in human subjects *in vivo* with low variability for prolonged durations. In addition, the present inventors could demonstrate that dOFM has the necessary accuracy and reproducibility to confirm BE for a reference acyclovir cream 5% compared to itself, and is sufficiently sensitive to discriminate inequivalent BA between two different topical acyclovir cream 5% products, in both cases based upon conventional BE criteria and pharmacokinetic (PK) endpoints. In this experiment, the physiological salt solution as described herein (see, for example, section 1) was used. This proprietary physiological salt solution comes the composition of dermal interstitial fluid very close and, thus, is able to avoid complex exchange processes at the exchange area of dOFM and dMD.

## Claims

1. A physiological salt solution comprising serum albumin, wherein the serum albumin is comprised in a concentration of between 10 and 30 g/l.

2. The physiological salt solution of claim 1, wherein the salt solution further comprises alpha-1-acid glycoprotein, wherein the alpha-1-acid glycoprotein is preferably comprised in a concentration of between 0.005 and 0.4 g/l.

3. The physiological salt solution of claims 1 or 2, wherein the salt solution further comprises immunoglobulin G, wherein the immunoglobulin G is preferably comprised in a concentration of between 1×10⁻⁷ and 4×10⁻⁴ g/l.

4. The physiological salt solution of any one of claims 1 to 3, wherein the salt solution further comprises deuterated water, wherein the deuterated water is preferably comprised in a concentration of between 0.15 and 500 ml/l.

5. The physiological salt solution of any one of claims 1 to 4, wherein the salt solution comprises
serum albumin and alpha-1-acid glycoprotein, or
serum albumin and immunoglobulin G, or
serum albumin, alpha-1-acid glycoprotein, and immunoglobulin G, or
serum albumin and deuterated water, or
serum albumin, alpha-1-acid glycoprotein, and deuterated water, or
serum albumin, immunoglobulin G, and deuterated water, or
serum albumin, alpha-1-acid glycoprotein, immunoglobulin G, and deuterated water.

6. The physiological salt solution of claim 5, wherein
serum albumin is comprised in a concentration of between 10 and 30 g/l,
alpha-1-acid glycoprotein is comprised in a concentration of between 0.005 and 0.4 g/l,
immunoglobulin G is comprised in a concentration of between 1×10⁻⁷ and 4×10⁻⁴ g/l, and/or deuterated water is comprised in a concentration of between 0.15 and 500 ml/l.

7. The physiological salt solution of any one of claims 1 to 6, wherein the salt solution further comprises one or more salts selected from the group consisting of sodium chloride (NaCI), potassium chloride (KCl), calcium chloride dihydrate (CaCl₂·2H₂O), magnesium chloride hexahydrate (MgCl₂·6H₂O), sodium acetate trihydrate (CH₃COONa·3H₂O), and sodium caprylate.·

8. Use of the physiological salt solution of any one of claims 1 to 7 as
dialysis solution for microdialysis (MD), or
perfusion solution for Open Flow Microperfusion (OFM).

9. A kit for microdialysis (MD) or Open Flow Microperfusion (OFM), said kit comprises:
(i) a physiological salt solution of any one of claims 1 to 7,
(ii) a dialysis or perfusion tube, and
(iii) optionally additional means for carrying out microdialysis (MD) or Open Flow Microperfusion (OFM).

10. A method for determining bioequivalence between a test substance and a reference substance using a kit of claim 9.

11. The method of claim 10, wherein the method comprises the steps of:
(i) providing at least one dialysate or perfusate comprising a test substance obtained from an individual treated on its skin with the test substance,
(ii) providing at least one dialysate or perfusate comprising a reference substance obtained from the individual treated on its skin with the reference substance,
(iii) determining at least one pharmacokinetic parameter of the test substance in the at least one dialysate or perfusate provided in (i) and determining (the same) at least one pharmacokinetic parameter of the reference substance in the at least one dialysate or perfusate provided in (ii), and
(iv) comparing the at least one pharmacokinetic parameter of the test substance with the (same) at least one pharmacokinetic parameter of the reference substance, wherein an equivalence/a comparability between the at least one pharmacokinetic parameter of the test substance with the (same) at least one pharmacokinetic parameter of the reference substance indicates bioequivalence of the test substance and the reference substance.

12. The method of claims 10 or 11, wherein
the dialysate comprising a test substance or reference substance further comprises the physiological salt solution of any one of claims 1 to7 and dermal interstitial fluid, or
the perfusate comprising a test substance or reference substance further comprises the physiological salt solution of any one of claims 1 to 7 and dermal interstitial fluid.

13. A method for determining bioequivalence between a first treatment site of an individual treated with a test substance and a second treatment site of the individual treated with the (same) test substance using a kit of claim 9.

14. The method of claim 13, wherein the method comprises the steps of:
(i) providing at least one dialysate or perfusate comprising a test substance obtained from an individual treated on its skin at a first treatment site with the test substance,
(ii) providing at least one dialysate or perfusate comprising the (same) test substance obtained from the individual treated on its skin at a second treatment site with the (same) test substance,
(iii) determining at least one pharmacokinetic parameter of the test substance at the first treatment site in the at least one dialysate or perfusate provided in (i) and determining (the same) at least one pharmacokinetic parameter of the (same) test substance at the second treatment site in the at least one dialysate or perfusate provided in (ii), and
(iv) comparing the at least one pharmacokinetic parameter of the test substance at the first treatment site with the (same) at least one pharmacokinetic parameter of the (same) test substance at the second treatment site,
wherein an equivalence/a comparability between the at least one pharmacokinetic parameter of the test substance at the first treatment site with the (same) at least one pharmacokinetic parameter of the (same) test substance at the second treatment site indicates bioequivalence of the first treatment site of the individual treated with the test substance and the second treatment site of the individual treated with the (same) test substance.

15. The method of claims 13 or 14, wherein
the dialysate comprising a test substance further comprises the physiological salt solution of any one of claims 1 to 7 and dermal interstitial fluid, or
the perfusate comprising a test substance further comprises the physiological salt solution of any one of claims 1 to 7 and dermal interstitial fluid.
